# EUROPEAN PATENT APPLICATION

(11) **EP 4 159 864 A1**
(43) Date of publication of application: **05.04.2023**
(21) Application number: 21817428.2
(22) Date of filing: 31.05.2021
(51) Int. Cl.: C12N 15/70, C07K 14/005, C07K 14/195, A61K 39/12, A61P 31/12, C12N 15/62

(54) **RECOMBINANT EXPRESSION VECTOR FOR PRODUCING ENCAPSULIN-BASED VACCINE, AND PREPARATION METHOD THEREFOR**

(30) Priority: 01.06.2020 KR 20200066119; 12.05.2021 KR 20210061143
(71) Applicant: Inthera Inc., Seoul 05836 (KR)
(72) Inventor: CHOI, Deog Young, Seoul 06673 (KR); JUNG, Hyun Gyo, Seoul 04726 (KR)
(74) Representative: AWA Sweden AB
(86) International application number: PCT/KR2021/006758
(87) International publication number: WO 2021/246740

(57) **Abstract**

The present invention relates to an encapsulin protein and a fusion protein comprising the same, and more specifically to a recombinant expression vector for vaccine production, and a preparation method therefor, the vector comprising polynucleotides that encode a target protein, an encapsulin protein and an RNA interacting domain (RID) protein, so as to improve the expression efficiency of the target protein, and thus enables a water-soluble vaccine to be produced in a highly efficient manner and a large target protein to be used.

## Description

### CROSS-REFERENCE TO RELATED APPLICATION

This application claims priority to and the benefit of Korean Patent Application No. 10-2020-0066119, filed on June 1, 2020, and Korean Patent Application No. 10-2021-0061143, filed on May 12, 2021, the disclosures of which are incorporated herein by reference in their entirety.

### [Technical Field]

The present invention relates to an encapsulin protein and a fusion protein comprising the same for enhancing the expression efficiency of a target protein, and more specifically to a recombinant expression vector for vaccine production and a preparation method therefor, the vector comprising polynucleotides that encode a target protein, an encapsulin protein and an RNA interacting domain (RID) protein, so as to produce water-soluble vaccines in a highly efficient manner and to use large-sized target proteins.

### [Background Art]

Recently, the development of new prophylactic vaccines for efficient response to rapidly increasing infectious diseases and the development of therapeutic vaccines that help the human body fight against diseases that the body already has (viruses, cancers, *etc*.) are being actively conducted. Prophylactic vaccines are vaccines for the prevention of diseases targeting healthy people, and refer to vaccines that induce an immune response by injecting a pathogen. Therapeutic vaccines are vaccines that strengthen the immune system (self-antigen injection, *etc*.) for the treatment of disease in patients, and a lot of research and development are in progress as therapeutic vaccines have the potential to be therapeutic agents for cancer, Alzheimer's Disease and the like.

Traditional vaccines have been prepared by using attenuated strains whose toxicity is weakened through long-term subculture or inactivated strains through compound treatment based on a process of culturing the pathogen itself. However, in this case, there are disadvantages in that the development and manufacturing period is too long and it cannot be applied to the development of vaccines against pathogens that are difficult to culture.

In order to manufacture more effective vaccines without being affected by the culture of pathogen itself, subunit vaccines have been developed that use only specific proteins or denatured toxins among pathogen components as antigens. In this case, although it has the advantage of securing higher safety, it also has the disadvantage of requiring multiple administrations and a separate immune adjuvant due to relatively low immunogenicity.

For the rapid development of a vaccine with a safer and higher efficacy against rapidly increasing new variant virus infectious diseases, the virus-like particle (VLP) vaccine manufacturing method has recently been developed, and it is in the limelight as a next-generation vaccine manufacturing technique that can overcome the limitations of existing vaccine production technologies based on the direct production of infectious viruses by using cultured cells.

A virus-like particle (VLP) is a very complex and sophisticated structure in which a viral structural protein is specifically expressed to show a structure similar to that of a wild-type virus. It has a size of 20 to 100 nm in diameter and can be produced through virion assembly after specifically expressing the structural proteins of viruses whose sequences are already known by using various types of recombinant protein expression systems.

Although it has the same appearance as the actual infectious virus, it has no virus-derived genes, and thus, it is impossible to multiply, but it induces high immunogenicity through high-density & highly ordered conformation. It has the advantage of stimulating both the T-cell and B-cell immune pathways in the body because its structure is similar to that of a wild-type virus. In addition, since there is no genetic material in the formed structure and it has no infectivity, it has high safety and excellent structural stability. However, VLPs that have been approved as commercial vaccines are only a few cases because of their complex structure and very different characteristics for each virus, resulting in a complicated manufacturing process or low productivity.

For the production of a more improved VLP-based vaccine, the chimeric VLP manufacturing technique in which an external pathogen antigen is introduced into a VLP structure that forms a polymer structure well, has been developed and has been utilized to develop vaccines targeting various pathogens. It takes the form of expressing external pathogen antigens on the surface of a VLP-based structure composed of structural proteins or self-assembling proteins of other specific viruses. In order to introduce a foreign antigen into the VLP-based structure, the chemical conjugation or genetic fusion method is used. The chemical conjugation method is to chemically fuse a separately prepared external antigen to an already prepared VLP-based structure through covalent bonding, and it has been pointed out that there is difficulty in arranging antigens in a certain direction and that the density of expressed antigens is relatively low. On the other hand, in the case of the genetic fusion method, the gene of a VLP structural protein and the gene of a target antigen protein are fused and expressed at the DNA level such that it is possible to produce a chimeric VLP with a simpler process. However, in this case, only very small foreign antigens composed of 10 to 20 amino acids or less can be introduced, and if they are larger than that, polymer formation by structural proteins is inhibited due to steric hindrance due to structural limitations. In particular, when expressed in *Escherichia coli* for high-efficiency and rapid production, proper protein folding is not performed, resulting in the formation of an insoluble inclusion body and making VLP production impossible.

The inventors of the present invention have shown that when the RNA interaction domain (RID) including the aminoacyl RNA synthetase N-terminal domain (ARSNTD) is used as a binding partner, it is involved in increasing protein folding and water solubility of various proteins

In the present invention, by using the RNA interaction domain (RID) as a novel fusion partner for more efficient and stable expression of a large-sized target antigen protein, which was previously impossible in the VLP structure made through the self-assembly of an encapsulin protein, the highly efficient and water-soluble expression of a fusion protein of encapsulin and target antigen is possible. The present invention was completed by confirming that the fusion protein makes it possible to rapidly produce a large amount of a fusion virus chimeric VLP through self-assembly after purification without the need for a refolding process.

### [Disclosure]

### [Technical Problem]

An object of the present invention is to provide a vector which is capable of more efficiently and stably expressing a large-sized target protein and is capable of the water-soluble expression of a fusion protein of an encapsulin protein and a target protein with high efficiency, and to provide a fusion protein which can be used to prevent or treat enteritis or cervical cancer by using a viral antigen protein, such as rotavirus, cervical cancer virus and the like, as a target protein.

Another object of the present invention is to provide a fusion protein that can be used to prevent or treat dengue fever caused by dengue virus or respiratory infections caused by coronavirus by using a virus antigen protein, such as dengue virus or coronavirus, as a target protein.

### [Technical Solution]

In order to solve the above-described problems, the present invention provides a recombination expression vector for vaccine production, including polynucleotides that encode an encapsulin protein and a target protein.

In addition, the present invention provides a recombination expression vector for vaccine production, including polynucleotides that encode a target protein, an encapsulin protein and an RNA interacting domain (RID) protein.

According to an exemplary embodiment of the present invention, the polynucleotide may further encode the TEV protein for cleavage of the RID protein.

In addition, the present invention provides a fusion protein, including a target protein; an encapsulin protein; and an RNA interacting domain (RID) protein.

According to an exemplary embodiment of the present invention, the target protein may be linked to the C-terminus of the encapsulin protein.

In addition, the present invention provides a method for producing a vaccine, including the steps of a) producing a recombinant expression vector for vaccine production including polynucleotides that encode a target protein; an encapsulin protein; and an RID protein, b) producing a transformant by introducing the recombinant expression vector into a host cell and c) culturing the transformant to induce the expression of a recombinant fusion protein, and obtaining the same.

According to an exemplary embodiment of the present invention, the RID protein may be used as a fusion partner for increasing the expression level or water solubility of the vaccine.

According to another exemplary embodiment of the present invention, the RID protein may include the amino acid sequence of SEQ ID NO: 12, SEQ ID NO: 13 or SEQ ID NO: 14 or a partial sequence thereof.

According to still another exemplary embodiment of the present invention, provided is a host cell which is transformed by the recombinant expression vector, and for the host cell according to the present invention, *E. coli* may be used.

According to an exemplary embodiment of the present invention, the encapsulin protein may include the amino acid sequence of SEQ ID NO: 1 or a partial sequence thereof.

According to another exemplary embodiment of the present invention, the target protein may be at least one protein selected from the group consisting of viral antigen protein, antibody, cell receptor, enzyme, structural protein, serum and cell protein.

According to still another exemplary embodiment of the present invention, the viral antigen protein may be cervical cancer virus antigen protein, Rotavirus antigen protein, coronavirus antigen protein or dengue virus antigen protein.

According to an exemplary embodiment of the present invention, at least one linker protein may be located between the target protein, the encapsulin protein and the RNA interacting domain (RID) protein, and wherein the linker protein may include gene sequence of SEQ ID NO: 15, SEQ ID NO: 16, SEQ ID NO: 17 or SEQ ID NO: 18.

### [Advantageous Effects]

The fusion protein prepared according to the present invention has the advantage of being able to more efficiently and stably express a large target protein.

According to an exemplary embodiment of the present invention, there is an advantage in that a fusion protein of an encapsulin and a target protein can be expressed in water-soluble form with high efficiency by including an RID as a novel fusion partner.

The fusion protein prepared according to the present invention has a structure similar to that of the wild-type virus and can stimulate both T-cell and B-cell immune pathways in the body, and thus has a high immune effect.

The fusion protein prepared according to the present invention may have a rotavirus antigen protein as a target protein, and enteritis can be prevented or treated by using the same.

In the fusion protein prepared according to the present invention, the target protein may be a dengue virus antigen protein, and dengue fever can be prevented or treated by using the same.

In the fusion protein prepared according to the present invention, the target protein may be a coronavirus antigen protein, and coronavirus infection can be prevented or treated by using the same.

In the fusion protein prepared according to the present invention, the target protein may be a cervical cancer virus antigen protein, and cervical cancer can be prevented or treated by using the same.

According to the vaccine production method of the present invention, it is possible to shorten the vaccine production time by rapidly producing a large amount of fusion protein.

### [Description of Drawings]

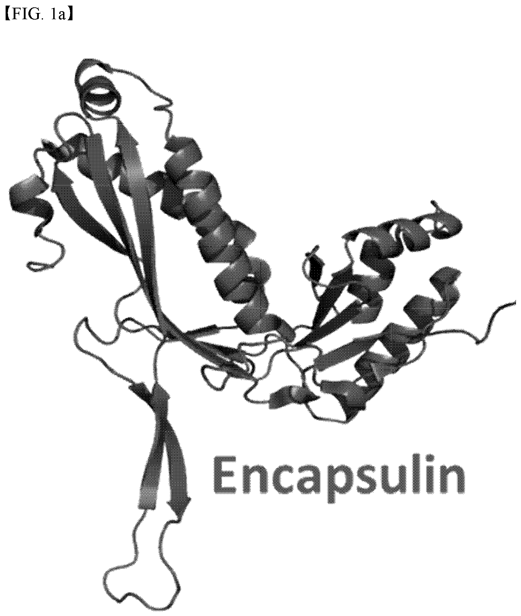

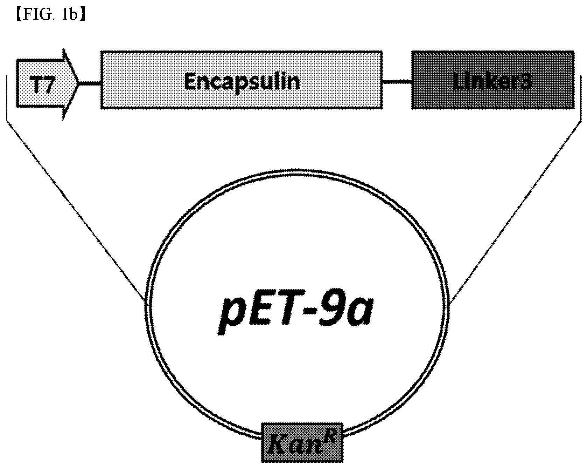

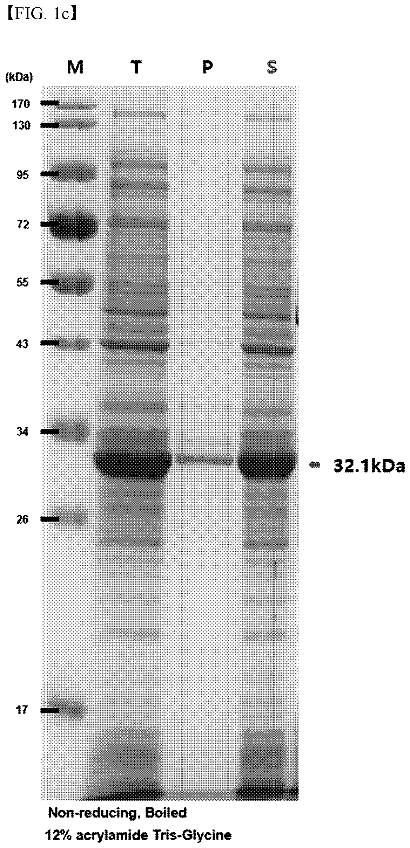

FIG. 1 is a mimetic diagram of the recombinant expression vector for producing encapsulin protein according to an exemplary embodiment of the present invention and the expression results of a highly efficient water-soluble protein. FIG. 1a is the protein structure of encapsulin predicted by using PDB according to an exemplary embodiment of the present invention. FIG. 1b is a mimetic diagram showing the structure of the pET9a-encapsulin recombinant expression vector according to an exemplary embodiment of the present invention. FIG. 1c is the result of confirming whether the high-efficiency water-soluble expression of the encapsulin protein occurred by SDS-PAGE using the expression vector.

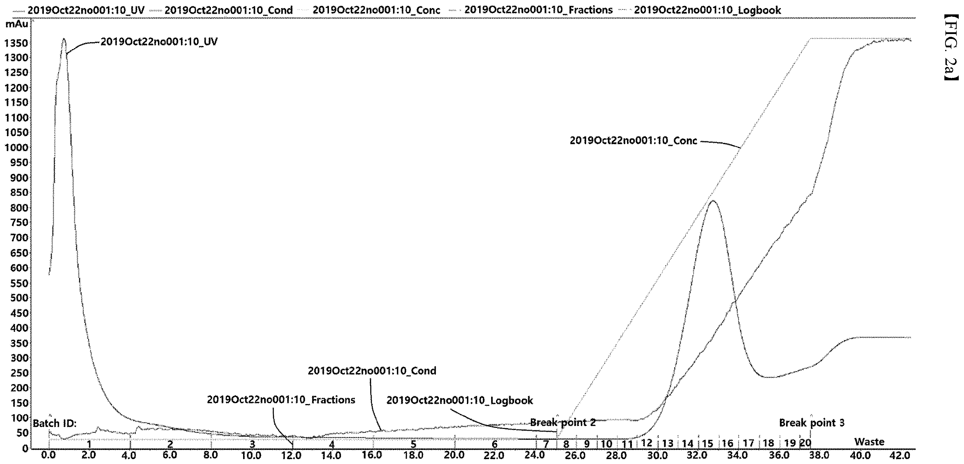

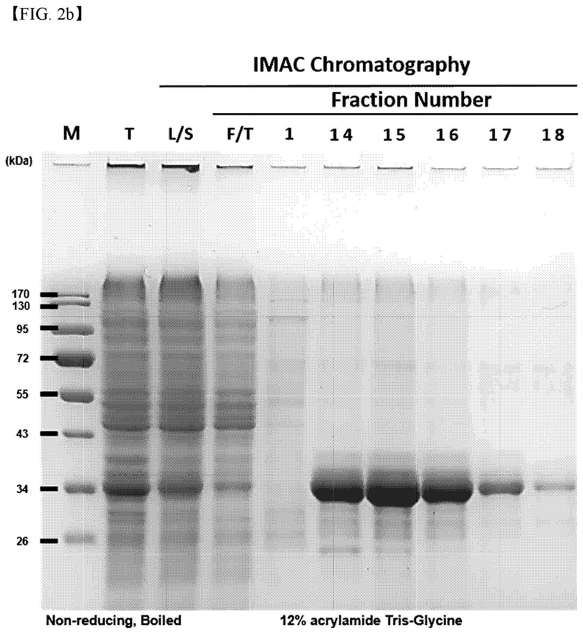

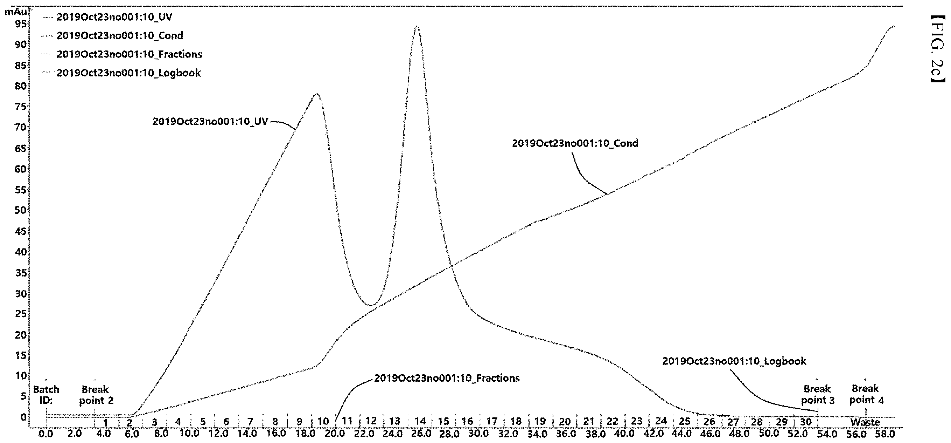

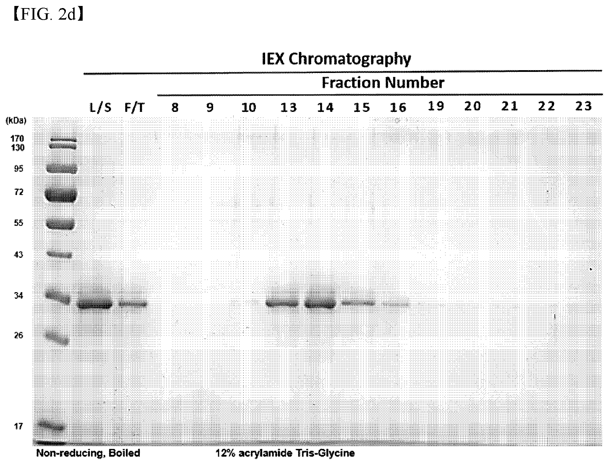

FIG. 2a is a chromatogram result when the encapsulin-linker protein expressed according to an exemplary embodiment of the present invention was purified through nickel affinity chromatography, and FIG. 2b is the result of confirming the separation pattern of the encapsulin-linker protein by SDS-PAGE. FIG. 2c is a chromatogram result when the encapsulin protein eluted from nickel affinity chromatography was purified through ion resin exchange chromatography, and FIG. 2d is the result of confirming the purification aspect of the encapsulin protein eluted from nickel affinity chromatography by SDS-PAGE.

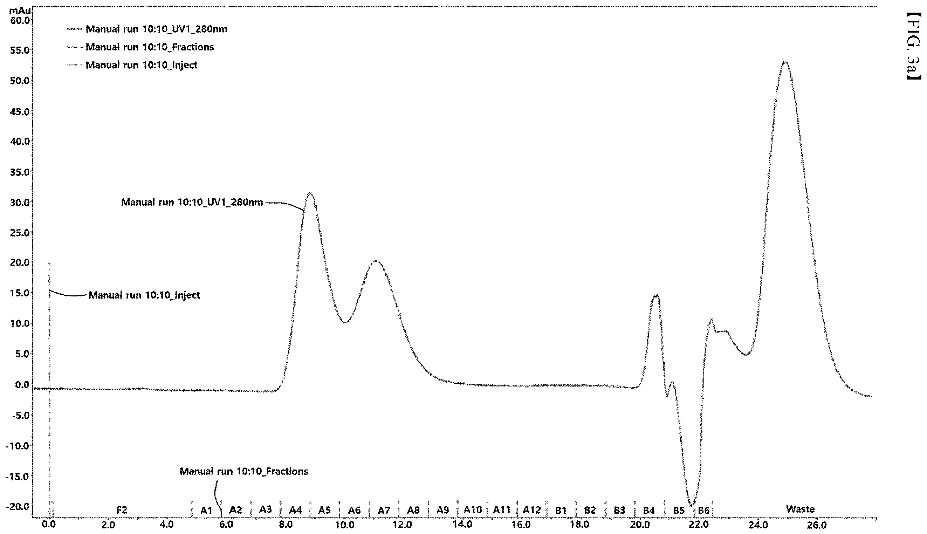

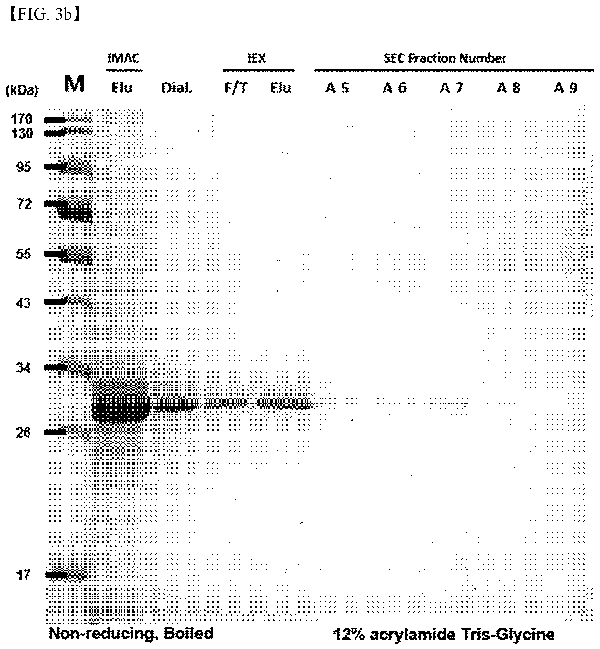

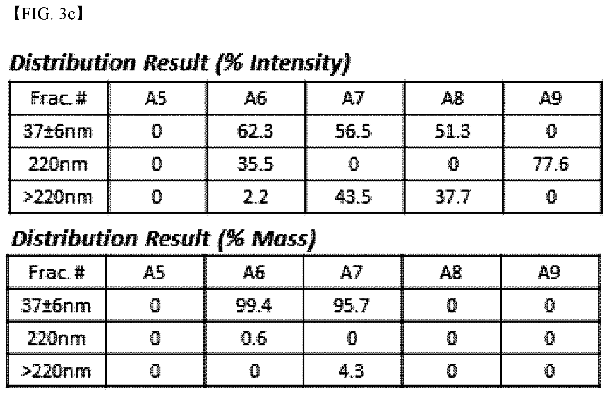

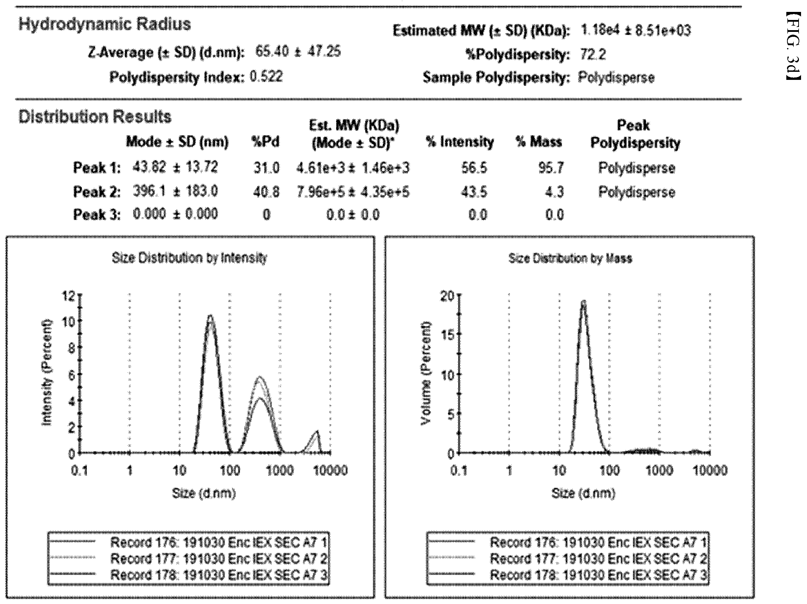

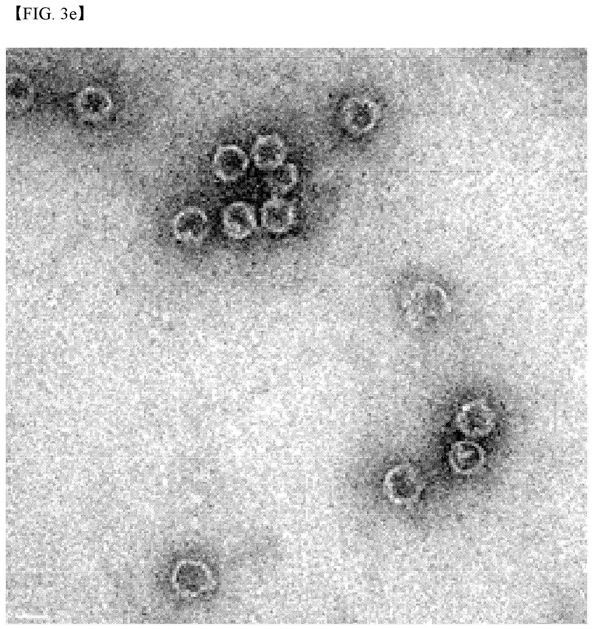

FIG. 3a is an elution graph after conducting size exclusion chromatography of a purified encapsulin protein. FIG. 3b is the result of confirming the separation pattern of the encapsulin protein after performing size exclusion chromatography by SDS-PAGE. FIGS. 3c and 3d are the results of analyzing the diameter distribution of the purified encapsulin protein particles through dynamic light scattering (DLS). FIG. 3e is the result of confirming the overall appearance of the purified encapsulin-based virus-like particle (VLP) through transmission electron microscopy (TEM).

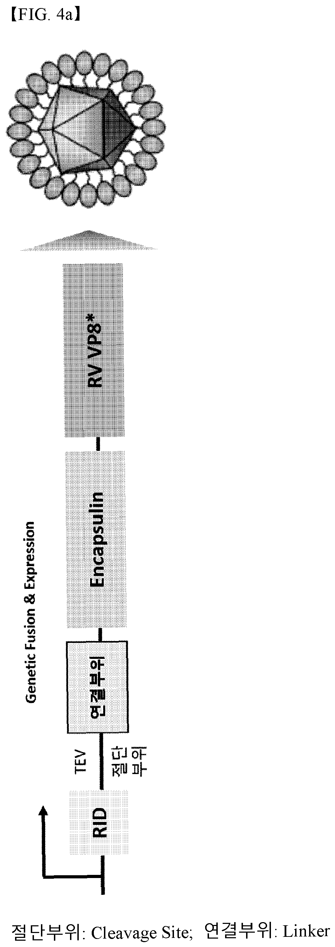

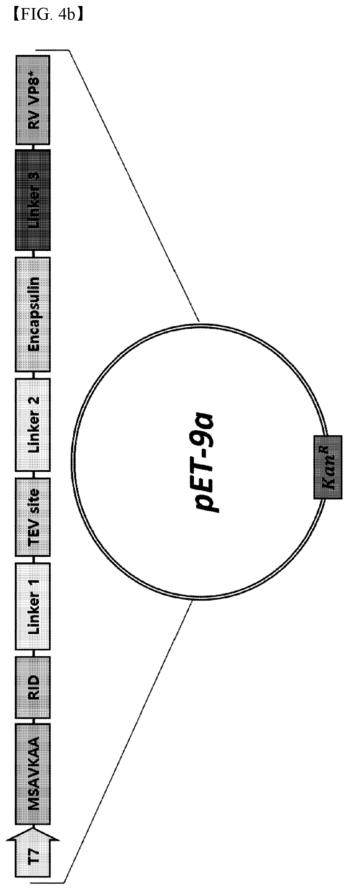

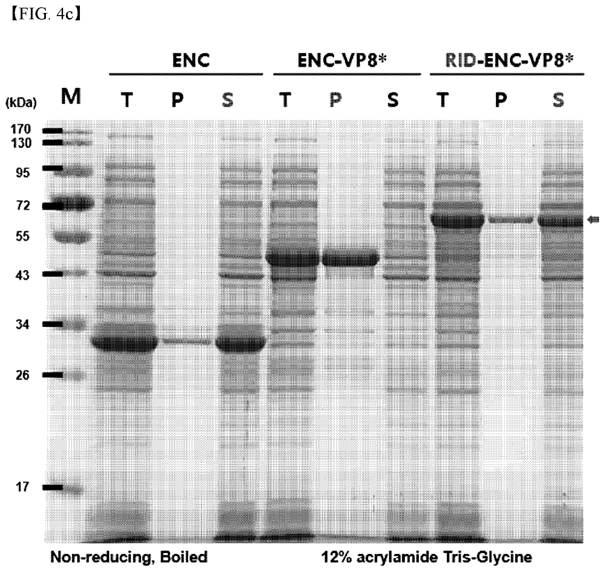

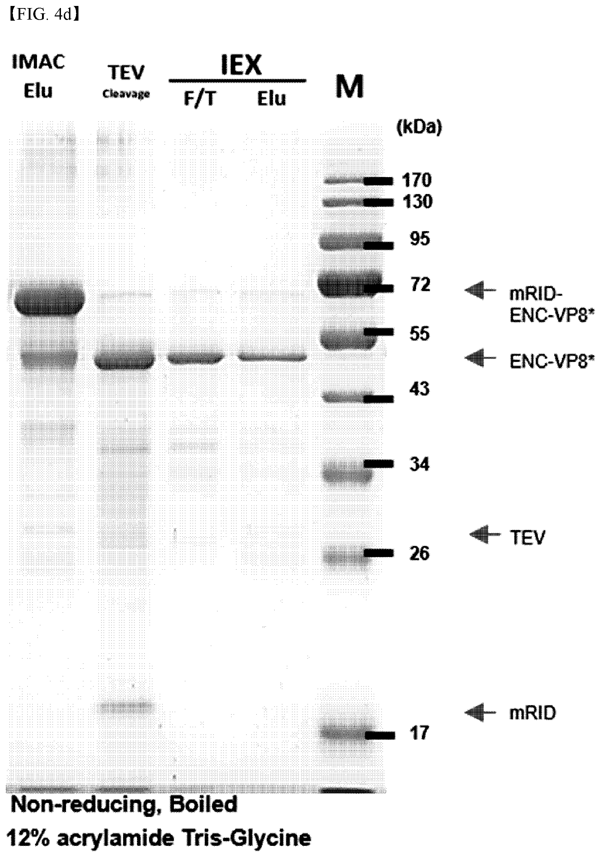

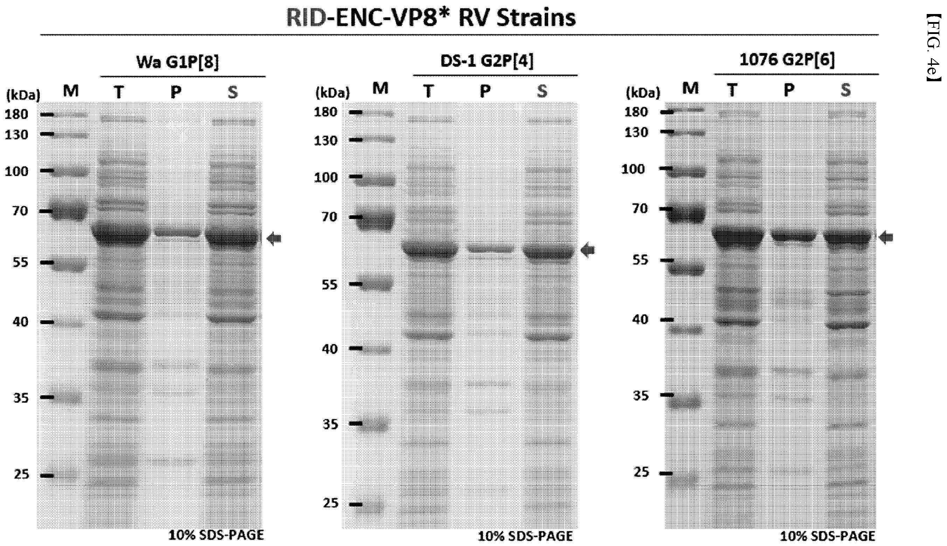

FIG. 4a illustrates a strategy for expressing an enzyme cleavage site and linker of RID, which is an expression level and water solubility enhancing fusion partner, to the encapsulin RV VP8* fusion protein, in order to prepare encapsulin-based virus-like particle expressing rotavirus VP8* according to an exemplary embodiment of the present invention. FIG. 4b shows the structure of the expression vector corresponding to FIG. 4a, and corresponds to the vector structure of pET9a-MSAVKAA-RIDLinker-TEV-Linker-Encapsulin-Linker, which was inserted with additional sequences for the efficiency of MSAVKAA-RID and TEV Cleavage, which are expression level and water solubility enhancement fusion partners. FIG. 4c shows SDS-PAGE results showing the effects of the fusion protein of encapsulin protein and antigen protein on fusion expression when RID, which is a fusion partner for enhancing expression level and water solubility, is applied to the peptide of the present invention. Relative changes in the expression levels of encapsulin and Encapsulin-Linker-VP8* can be confirmed, and it is the expression result of Encapsulin-Linker-VP8* recombinant protein (size: 50.3kDa), in which a protein in which the Encapsulin-Linker protein (size 32.1kDa) is recombined and VP8*, which is an antigen of rotavirus (RV) to confirm its role as a nano-platform, are bound to the C-terminus. In the case of FIG. 4d, it is the result of confirming the separation pattern and purity of protein through SDS-Page upon ion exchange resin purification after the TEV cleavage site applied between RID and encapsulin according to an exemplary embodiment of the present invention was treated with TEV enzyme. FIG. 4e shows the result of confirming the expression pattern for each type of recombinant expression vector rotavirus for vaccine production prepared according to an embodiment of the present invention.

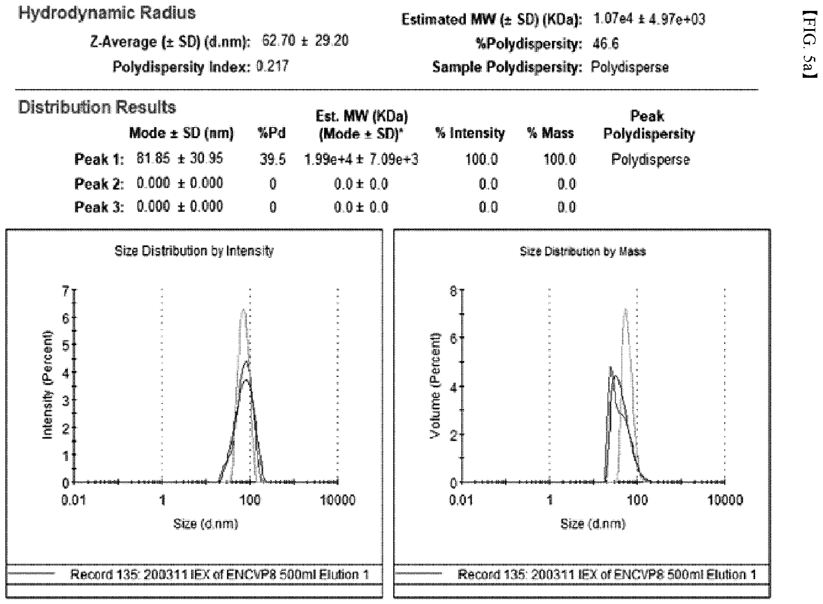

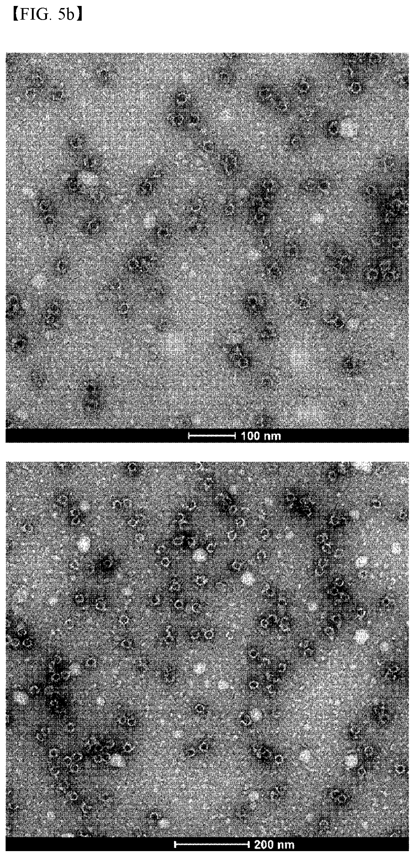

FIG. 5a is the result of analyzing the overall diameter distribution of VLPs formed by a fusion protein of purified encapsulin protein and antigenic protein through dynamic light scattering (DLS). FIG. 5b shows the results of confirming the overall appearance of an encapsulin-based virus-like particle (VLP) on which a target protein is expressed on the surface through a transmission electron microscope (TEM).

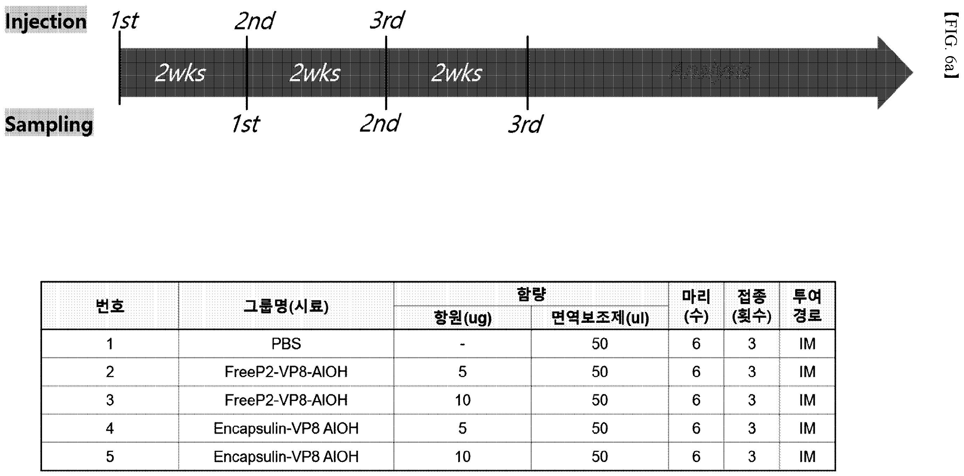

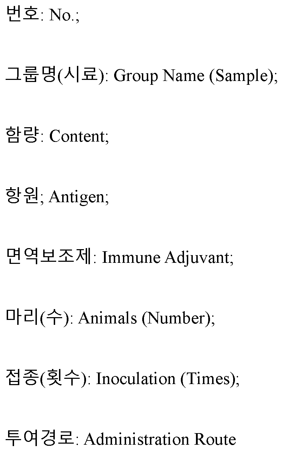

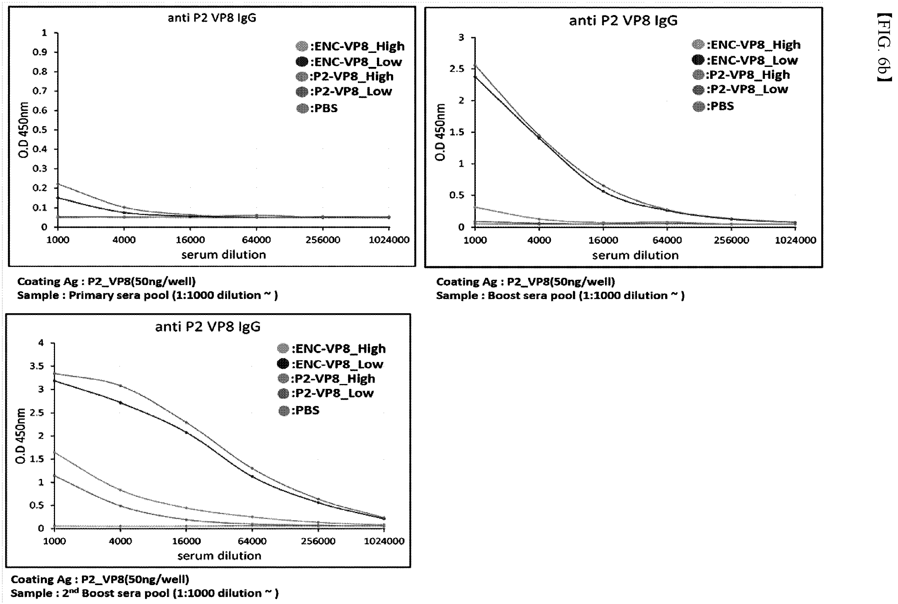

FIG. 6a is a schedule from the inoculation of samples according to an exemplary embodiment of the present invention sample into mice to serum acquisition, and a composition and content table of samples injected into mice according to an exemplary embodiment of the present invention. FIG. 6b shows the results from analyzing using the enzyme-linked immunosorbent assay (ELISA) to measure the antibody response specific to the target protein in serum obtained from an experimental group of animals inoculated with a VLP formed by a fusion protein of encapsulin and the target protein.

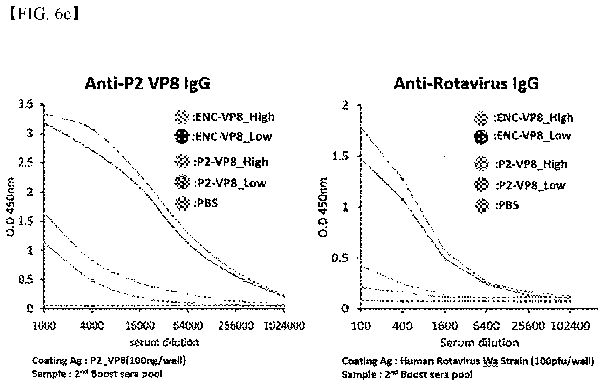

FIG. 6c shows the results of analyzing the target protein-specific antibody titer in serum obtained after inoculation with a VLP composed of the fusion protein of encapsulin and a target protein by enzyme-linked immunosorbent assay (ELISA). The first illustrated result is a result of adsorbing the recombinant protein P2-VP8* protein on an ELISA plate, and then measuring the specific antibody titer in the serum of the inoculated animal, and the second illustrated result is a result obtained after adsorbing Wa Strain itself, which is a human rotavirus, on an ELISA plate.

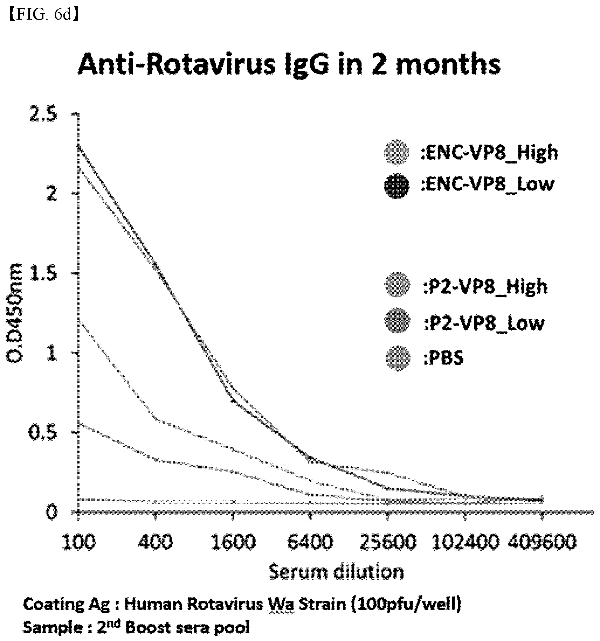

FIG. 6d is a measurement of the target protein-specific antibody titer in serum obtained after 2 months had elapsed after the inoculation of VLP composed of the fusion protein of encapsulin and a target protein three times, and first, this is the result of analyzing by enzyme-linked immunosorbent assay (ELISA) in which Wa Strain itself, which is a human rotavirus, was adsorbed on an ELISA plate and then the serum of the inoculated animal was reacted.

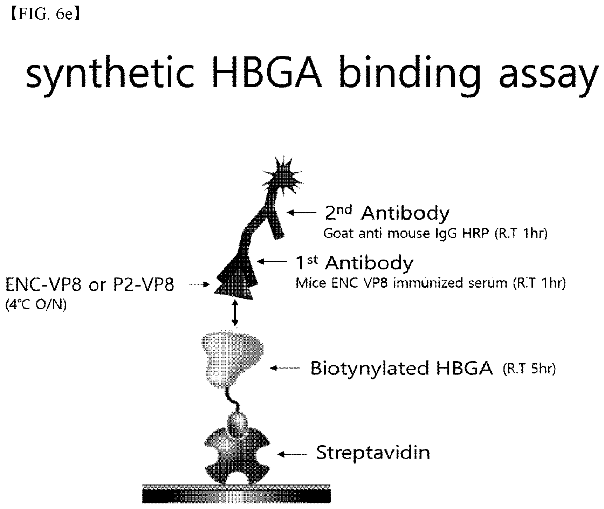

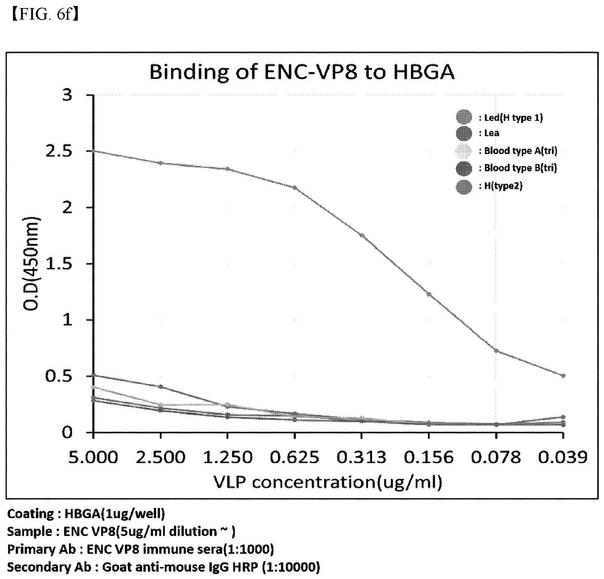

FIG. 6e illustrates the HBGA Binding Assay components and analysis process in order to measure the ability of a target protein to bind to HBGA, which is a receptor, and FIG. 6f shows the results of measuring the binding between the target protein and the receptor (Led (H type 1)).

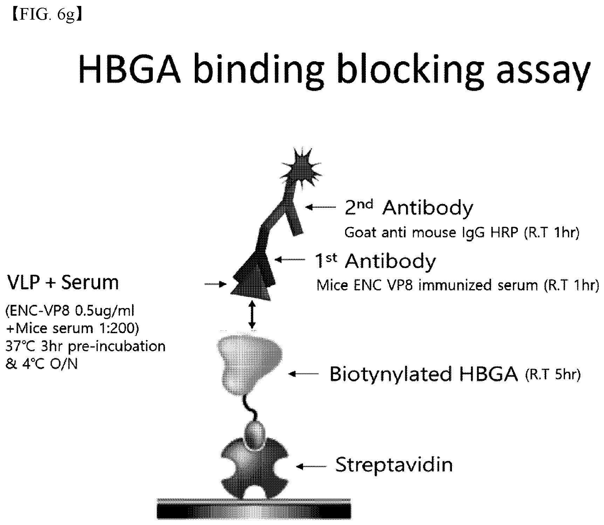

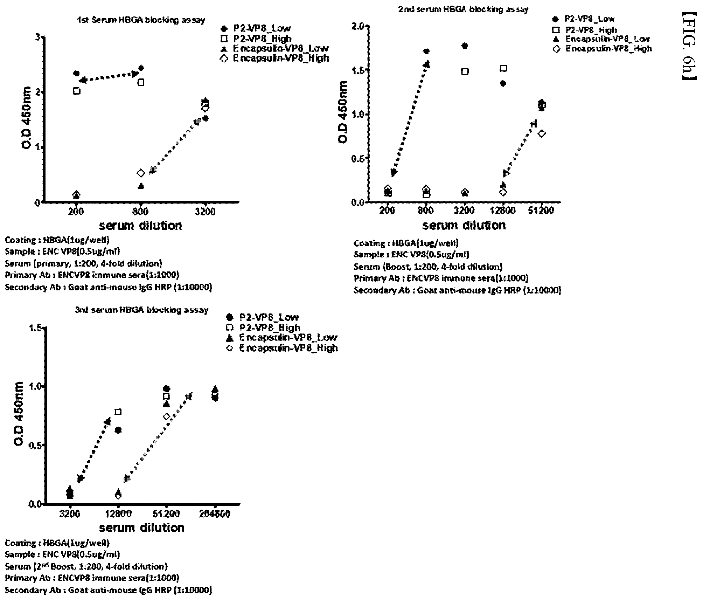

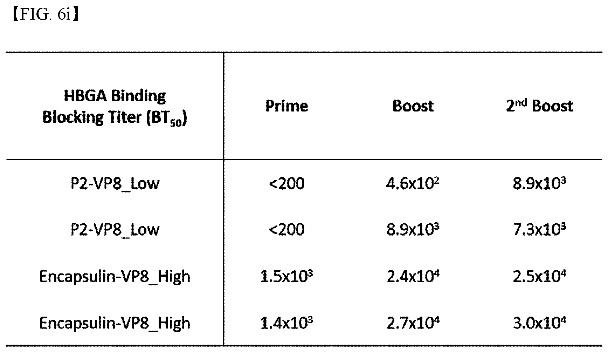

FIG. 6g illustrates the HBGA Blocking Assay components and the analysis process for measuring the ability of a target protein-specific antibody in the serum of an animal experiment group inoculated with a VLP formed by a fusion protein of encapsulin and a target protein to inhibit the binding between a target protein and a receptor, FIG. 6h shows the results of measuring the degree to which the binding between the target protein and the receptor (Led (H type 1)) is inhibited by immune serum, and FIG. 6i is values obtained by measuring the dilution factor (BT50) at which the immune serum of each experimental group inhibited the binding between the target protein and the receptor by 50%.

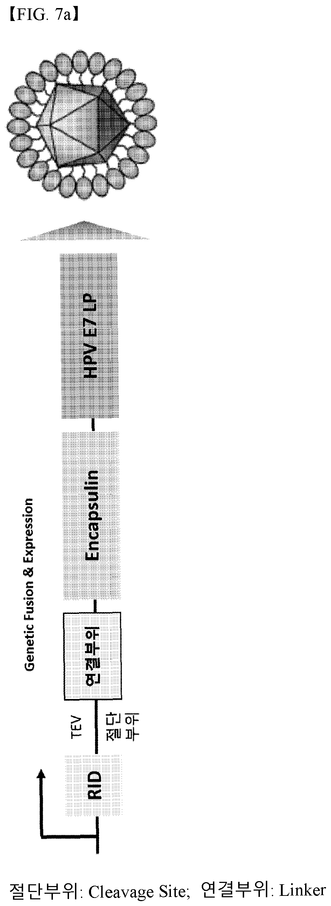

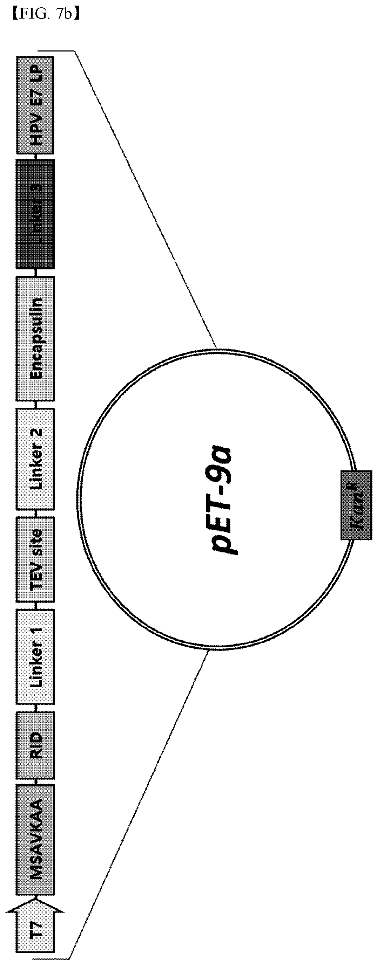

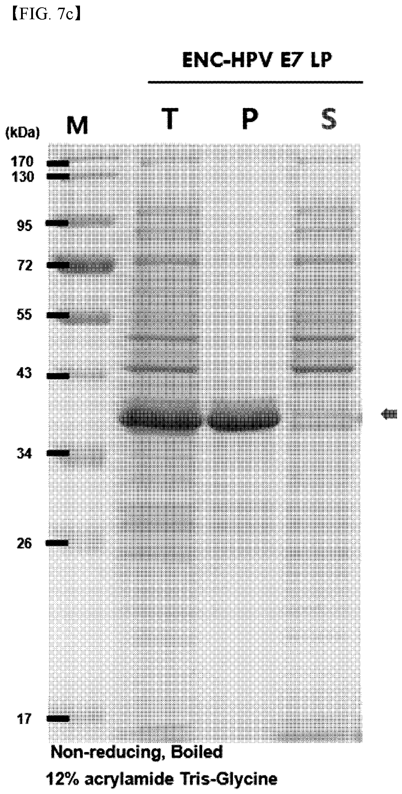

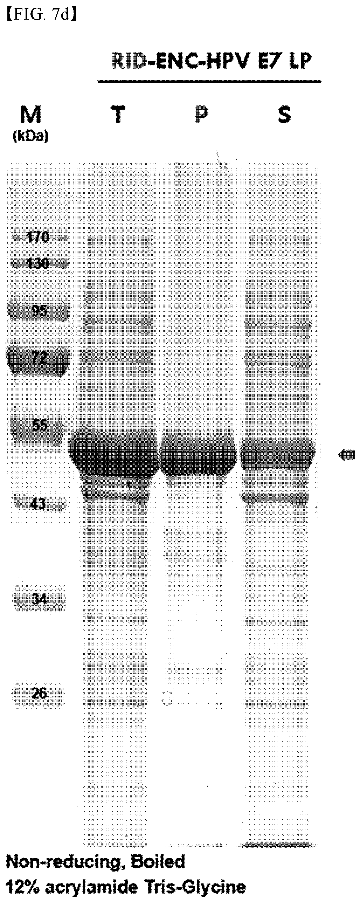

FIG. 7a is a mimetic diagram of polynucleotides encoding E7 LP, which is a peptide having the amino acid sequence of human papillomavirus, and an encapsulin protein according to an exemplary embodiment of the present invention, and a vaccine prepared thereby. FIG. 7b shows the structure of the expression vector corresponding to FIG. 7a, and images of the vector structure of pET9a-MSAVKAA-RID-Linker-TEV-Linker-Encapsulin, which was inserted with additional sequences for the efficiency of MSAVKAA-RID and TEV Cleavage, which are expression level and water solubility enhancement fusion partners. The effects on the fusion expression of the fusion protein of the encapsulin protein and the target protein when RID, which is a fusion partner for enhancing expression level and water solubility, were confirmed through the results of FIGS. 7c and 7d. FIG. 7c is an SDS-PAGE result of the Encapsulin-Linker-E7 recombinant protein (size: 36.1 kDa) in which E7 LP, which is an antigen of human papillomavirus (HPV) Type 16, was bound to the C-terminus. FIG. 7d is the result of confirming the changes in the water solubility and expression level of the protein by SDS-PAGE after RID was added to the N-terminus of Encapsulin-Linker-HPV E7 LP and then expressed. Through this, the necessity of RID, which is a fusion partner, was confirmed even when the human papillomavirus antigen was added to the C-terminus, as in the case of rotavirus.

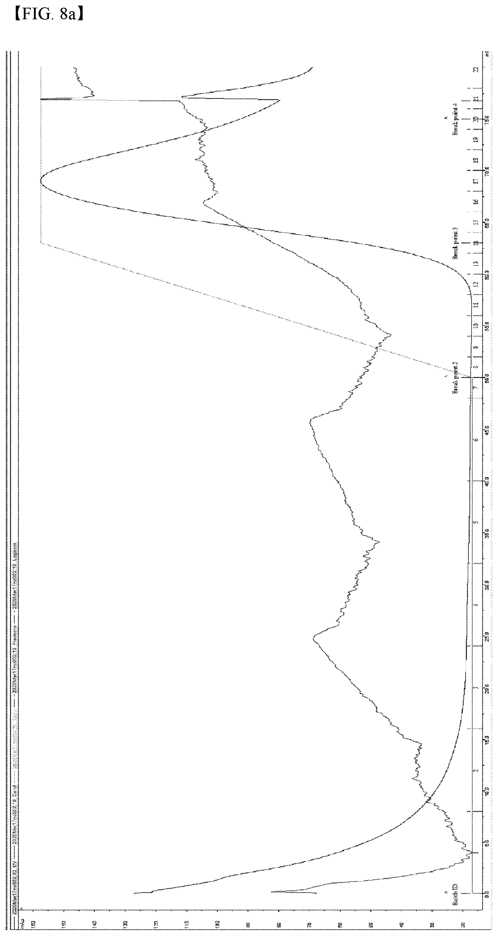

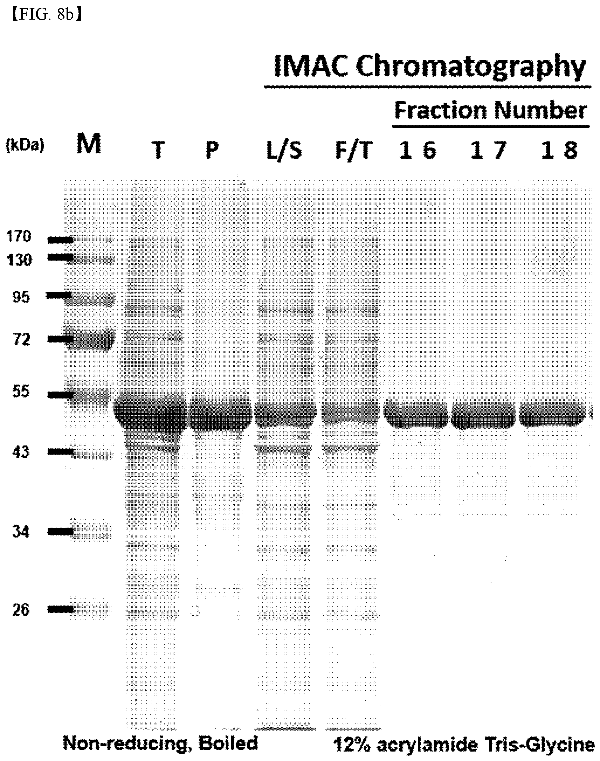

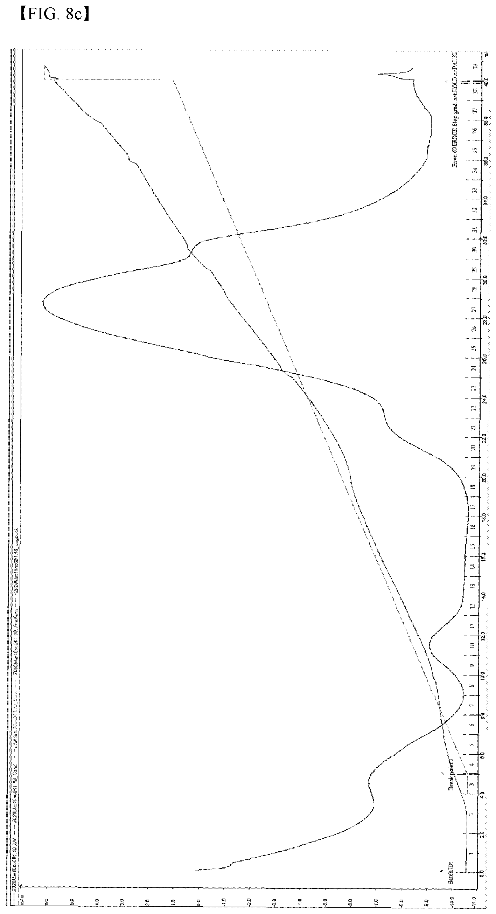

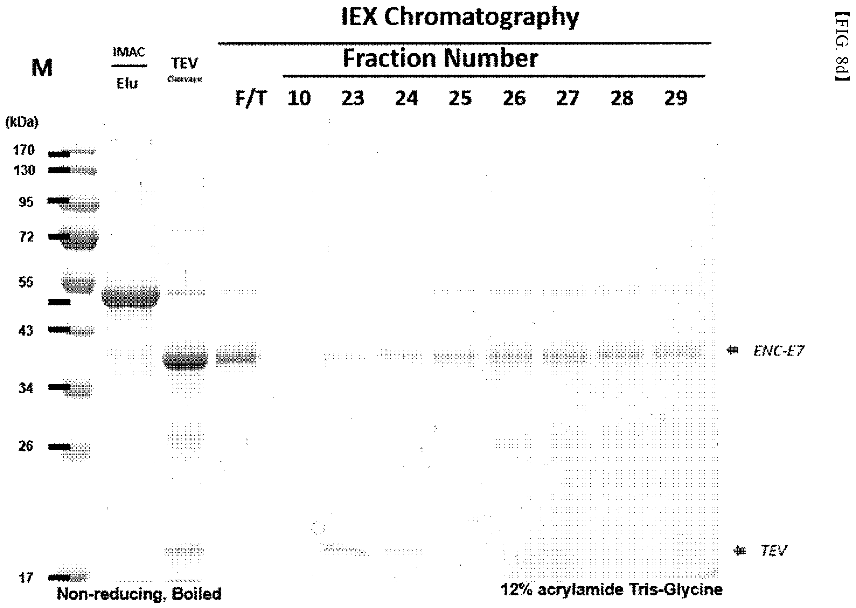

FIG. 8a is a chromatogram result when Encapsulin-Linker-HPV E7 LP protein expressed according to an exemplary embodiment of the present invention was purified through nickel affinity chromatography, and FIG. 8b is the result of confirming the separation pattern of the Encapsulin-Linker-HPV E7 LP protein by SDS-PAGE. FIG. 8c is a chromatogram result when the Encapsulin-Linker-HPV E7 LP protein fusion was purified through ion resin exchange chromatography in nickel affinity chromatography, and FIG. 8d is the result of confirming the purification aspect of the Encapsulin-Linker-HPV E7 LP protein by SDS-PAGE.

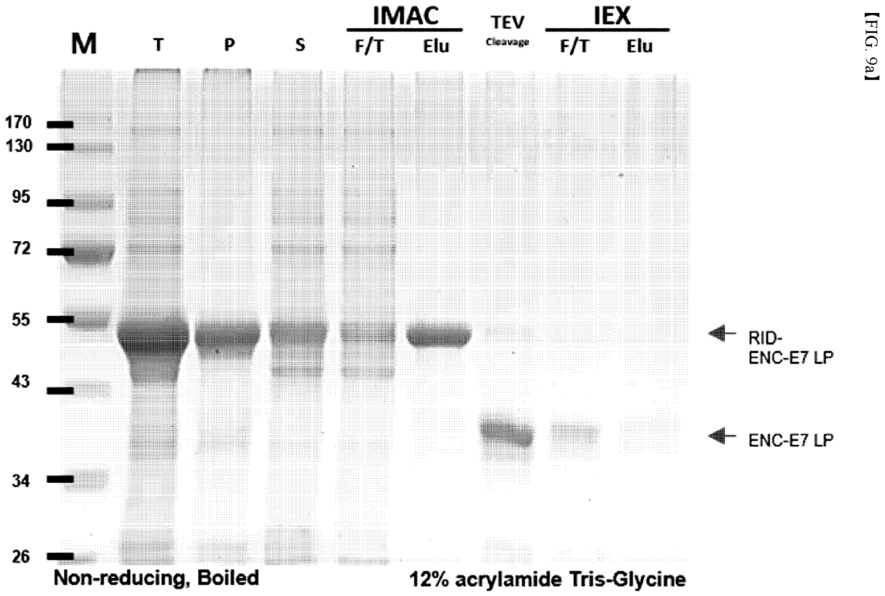

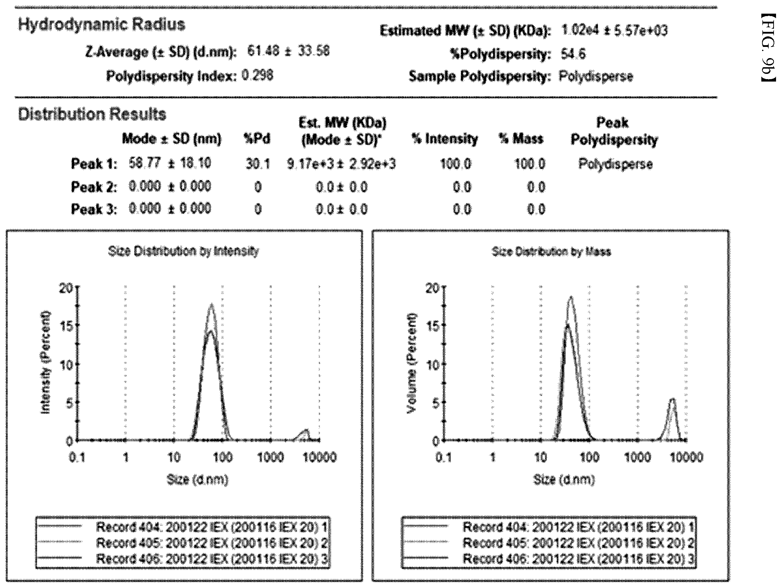

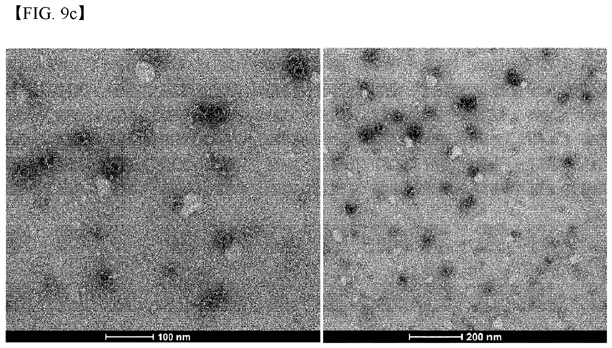

FIG. 9a is an SDS-PAGE result confirming the purification pattern and purity of the fusion protein in the purification process from the protein expression of Encapsulin-Linker-HPV E7 LP to TEV enzyme treatment and ion exchange resin chromatography conducted in the present invention. FIG. 9b shows the result of analysis through dynamic light scattering (DLS) to confirm the overall diameter distribution of the VLP of the purified encapsulin protein-target protein fusion. FIG. 9c shows the results of confirming the overall appearance of the VLP of the encapsulin-based fusion protein on which the target protein was expressed on the surface through a purified transmission electron microscope (TEM).

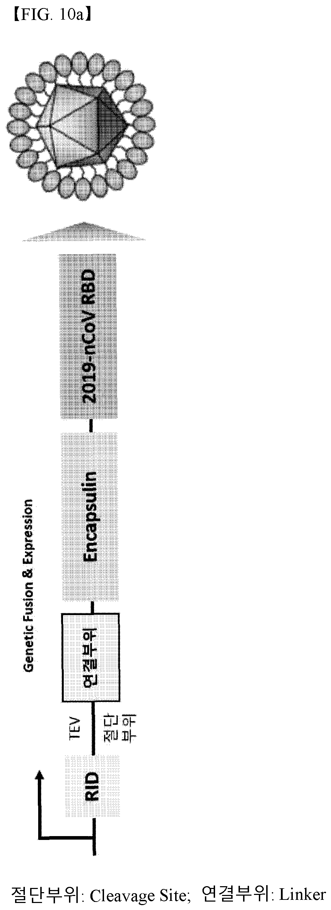

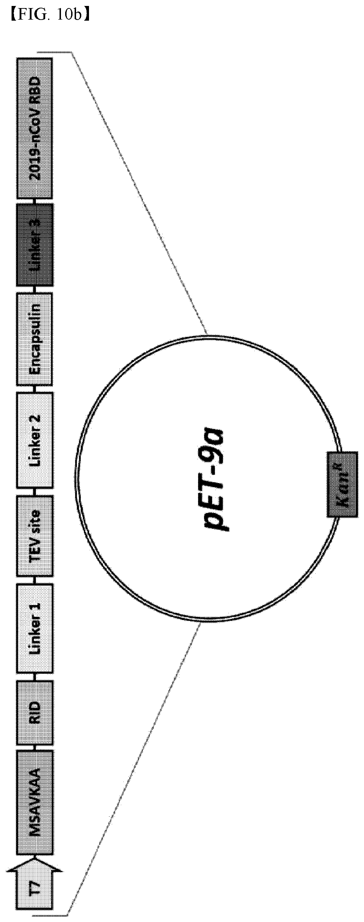

FIG. 10a illustrates a strategy for expressing an enzyme cleavage site and linker of RID, which is an expression level and water solubility enhancing fusion partner, to the encapsulin RV VP8* fusion protein, in order to prepare an encapsulin-based virus-like particle expressing coronavirus RBD antigen according to an exemplary embodiment of the present invention. FIG. 10b shows the structure of the expression vector corresponding to FIG. 10a, and corresponds to the vector structure of pET9a-MSAVKAA-RID-Linker1-TEV-Linker2-Encapsulin-Linker4-Coronavirus RBD, which was inserted with additional sequences for the efficiency of MSAVKAA-RID and TEV Cleavage, which are expression level and water solubility enhancement fusion partners.

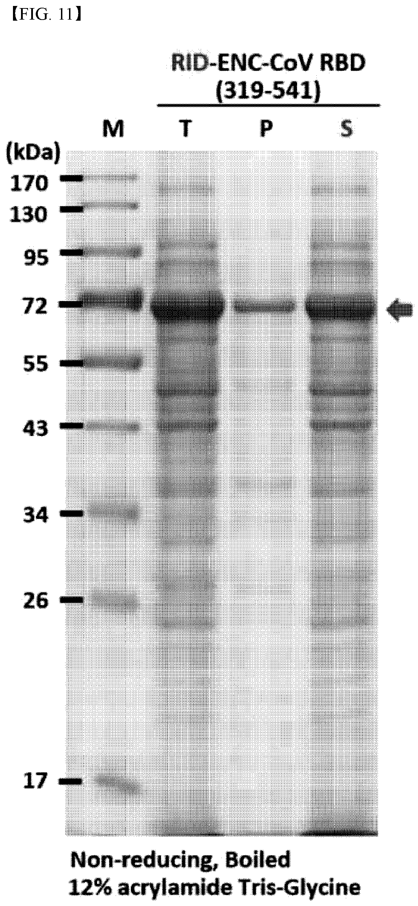

FIG. 11 is the result of confirming the effects of the fusion protein of encapsulin and the target protein on the expression level according to the application of RID, which is an expression level and a water-solubility enhancing fusion partner. In the case of a protein fused with RBD (size 23.6 kDa), which is an antigen of coronavirus (2019-nCoronavirus, CoV), to the C-terminus of encapsulin, the protein showed an insoluble expression pattern, whereas when the RID was fused to the N-terminus and expressed, it was confirmed through SDS-PAGE that most of them were expressed in water-soluble form.

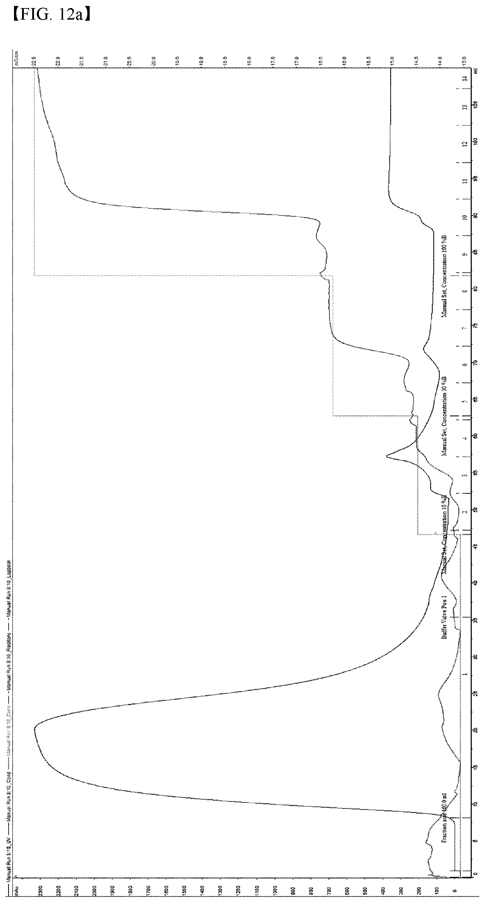

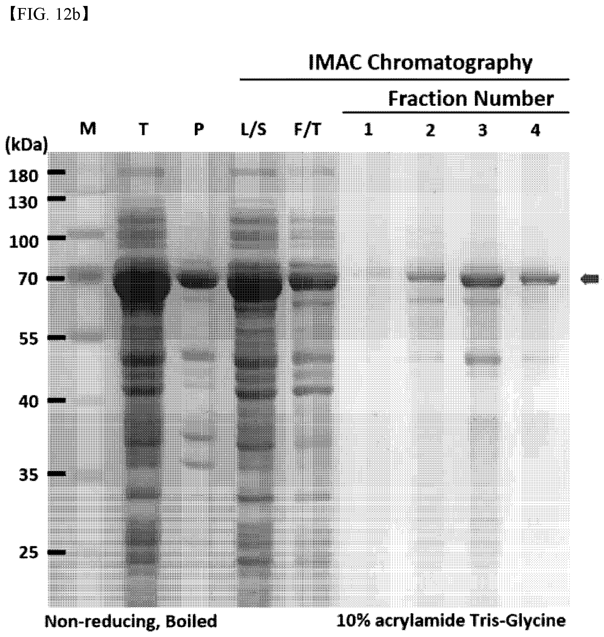

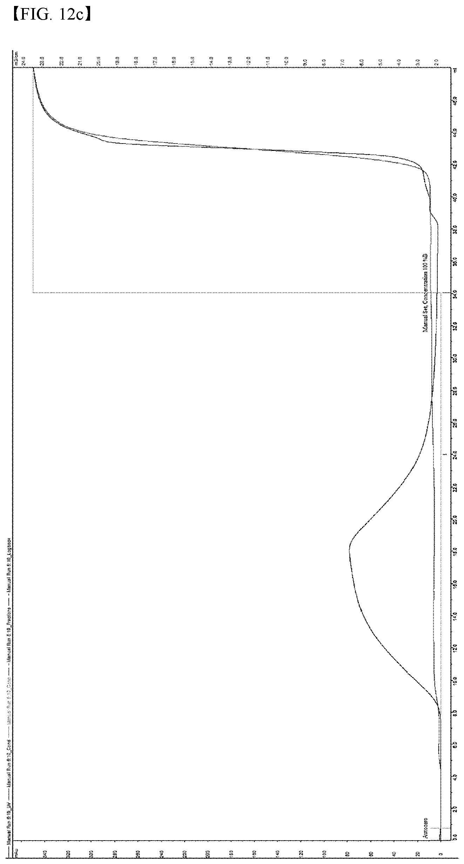

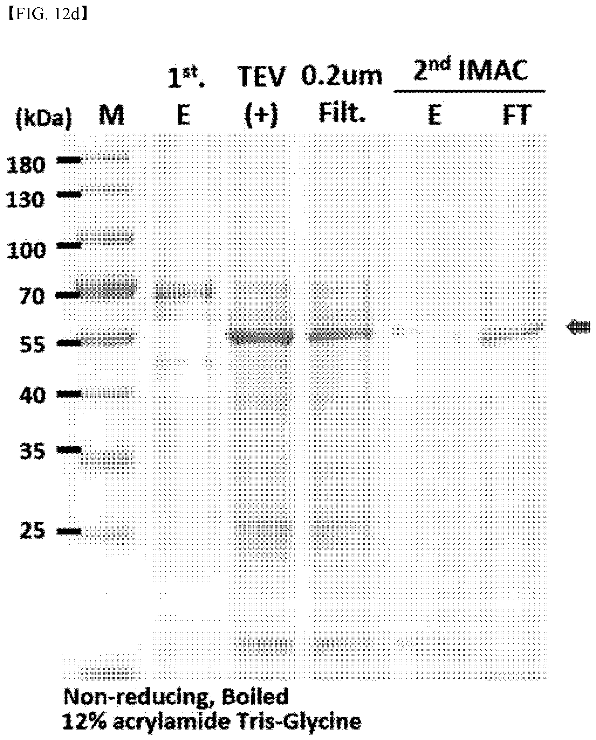

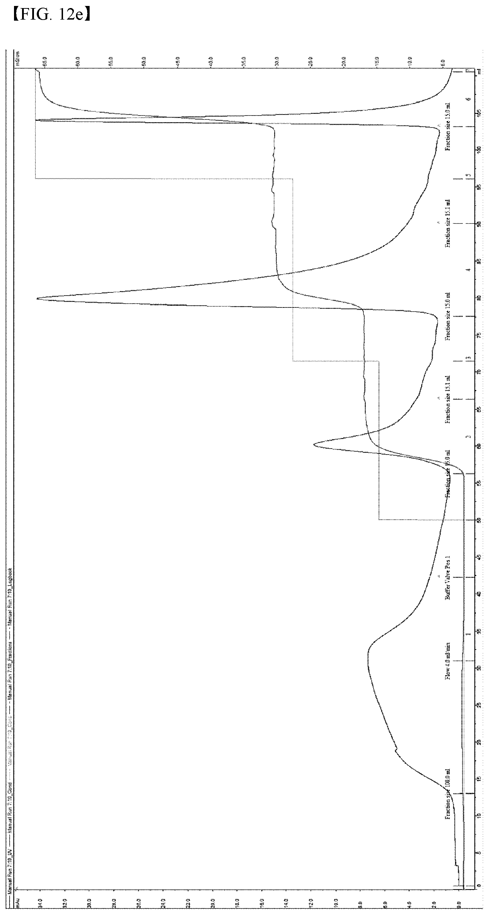

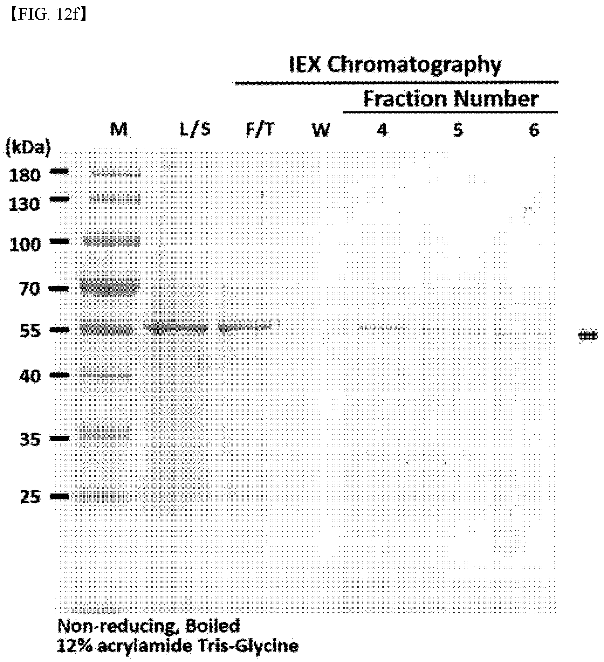

FIG. 12a is a chromatogram result when the RID-Encapsulin-Linker4-CoV RBD protein (size 66.4 kDa) expressed according to an exemplary embodiment of the present invention was purified through nickel affinity chromatography, and FIG. 12b is the result of confirming the separation pattern of RID-Encapsulin-Linker4-CoV RBD protein by SDS-PAGE. FIG. 12c is a chromatogram result when the Encapsulin-Linker4-CoV RBD protein fusion was purified by separating the N-terminal RID and the TEV cleavage site in nickel affinity chromatography, and FIG. 12d is an SDS-PAGE result confirming the purification pattern and purity of the fusion protein in the purification process ranging from TEV enzyme treatment and secondary nickel affinity chromatography of the Encapsulin-Linker4-CoV RBD protein. FIG. 12e is a chromatogram result when the Encapsulin-Linker4-CoV RBD protein fusion was purified through ion exchange resin chromatography in nickel affinity chromatography, and FIG. 12f is an SDS-PAGE result confirming the purification pattern and purity of the fusion protein.

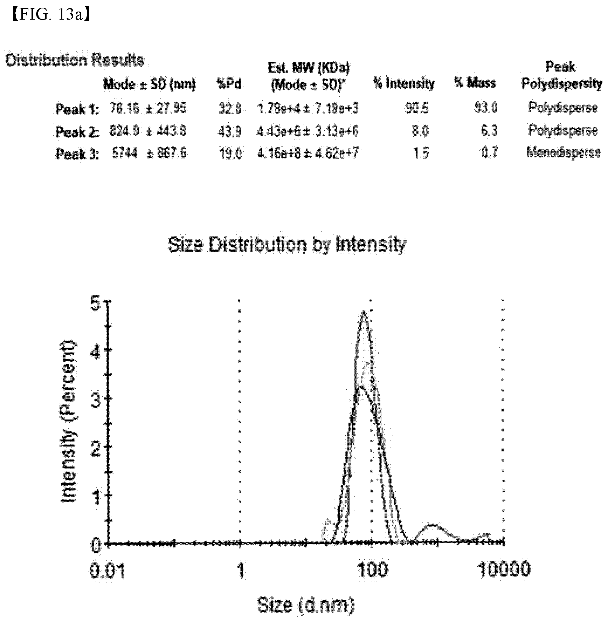

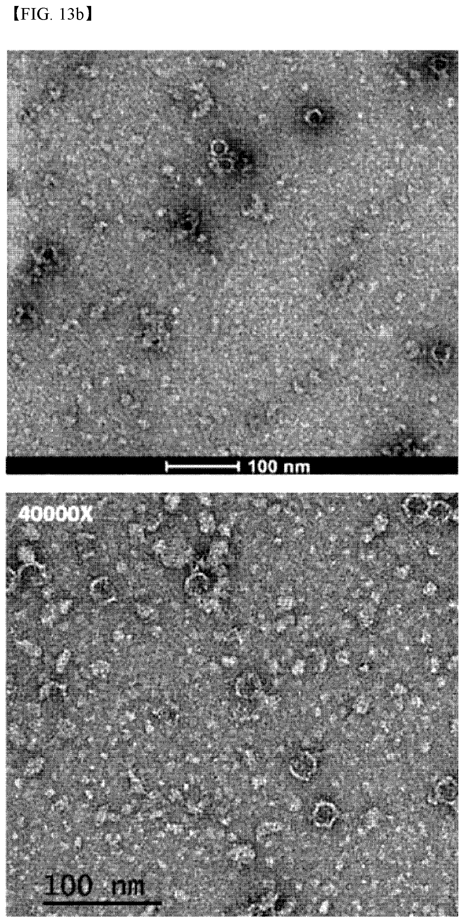

FIG. 13a shows the result of analysis through dynamic light scattering (DLS) to confirm the overall diameter distribution of the VLP of the purified encapsulin protein-target protein fusion. FIG. 13b shows the results of confirming the overall appearance of VLP of the encapsulin-based fusion protein with a target protein expressed on the surface through a purified transmission electron microscope (TEM).

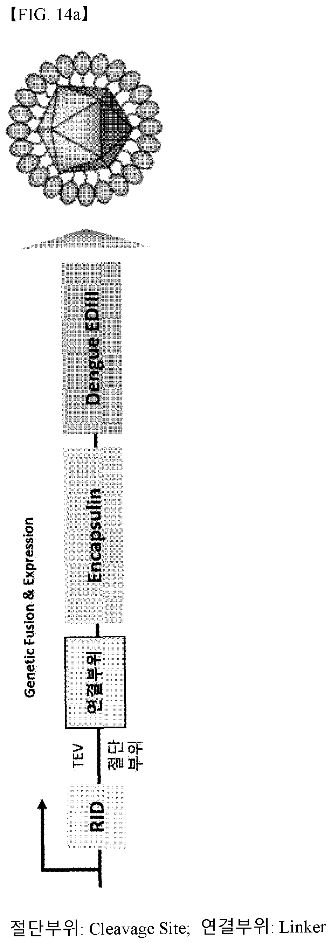

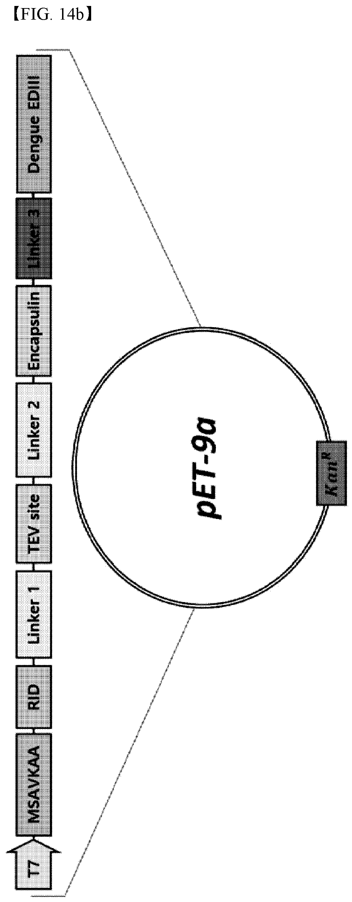

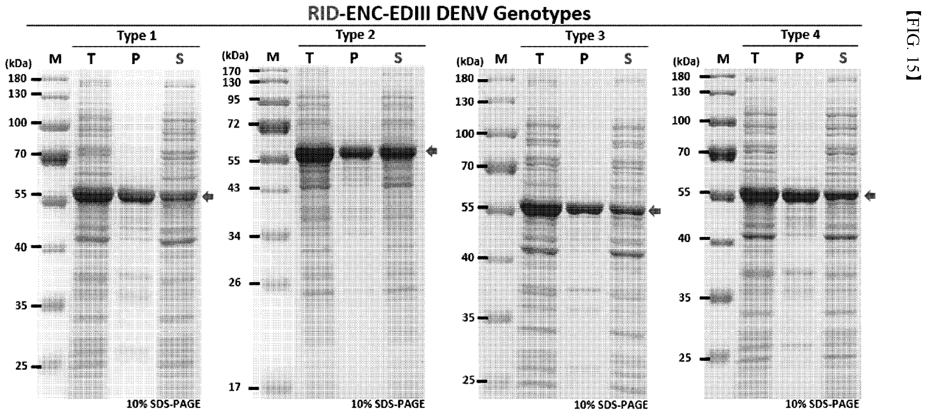

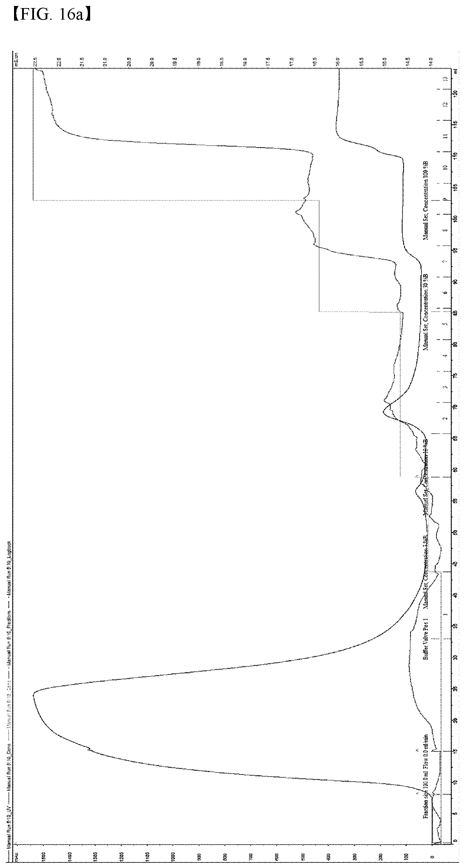

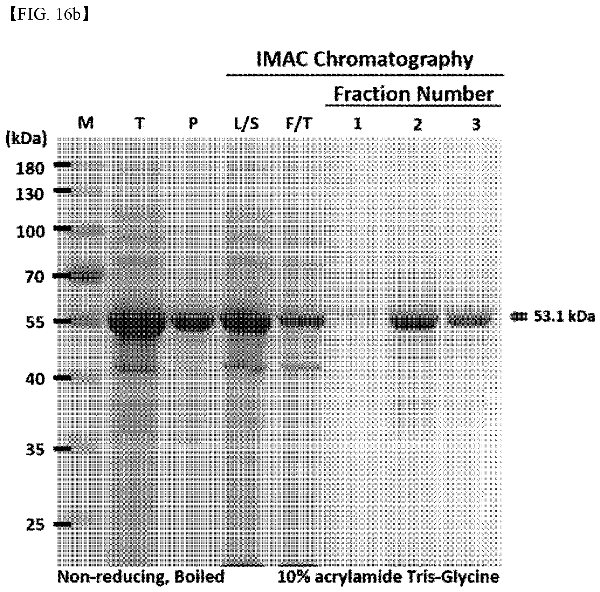

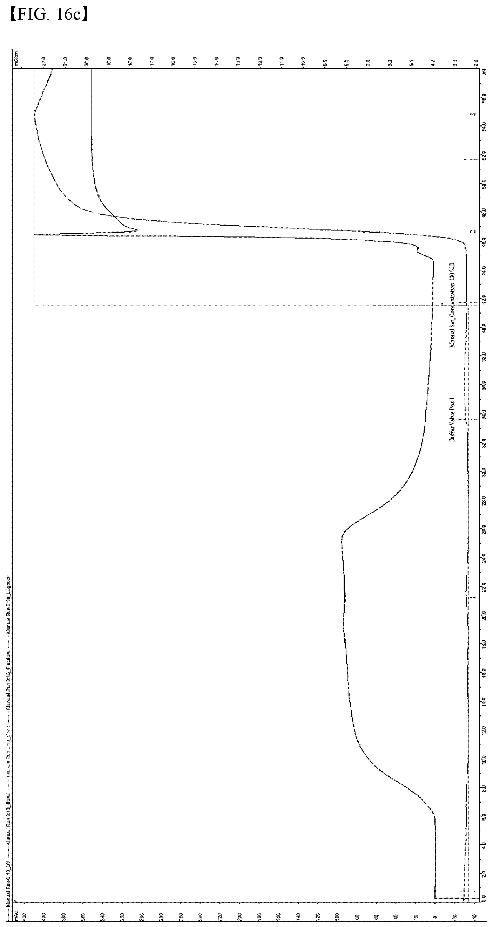

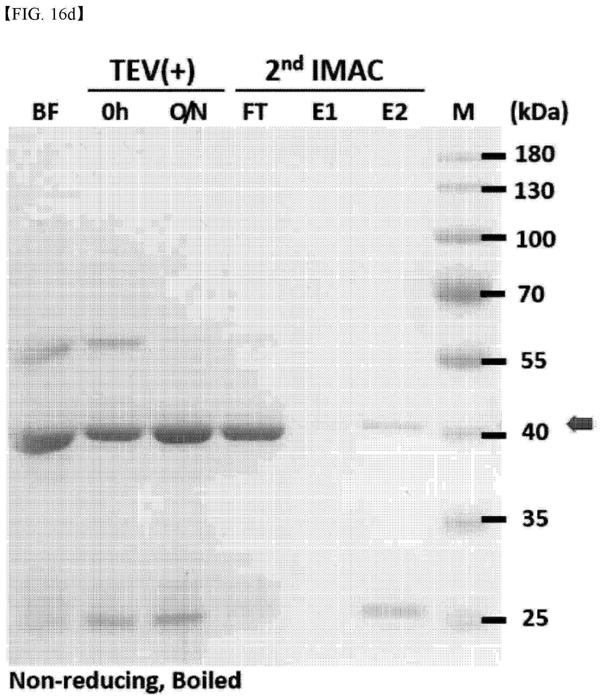

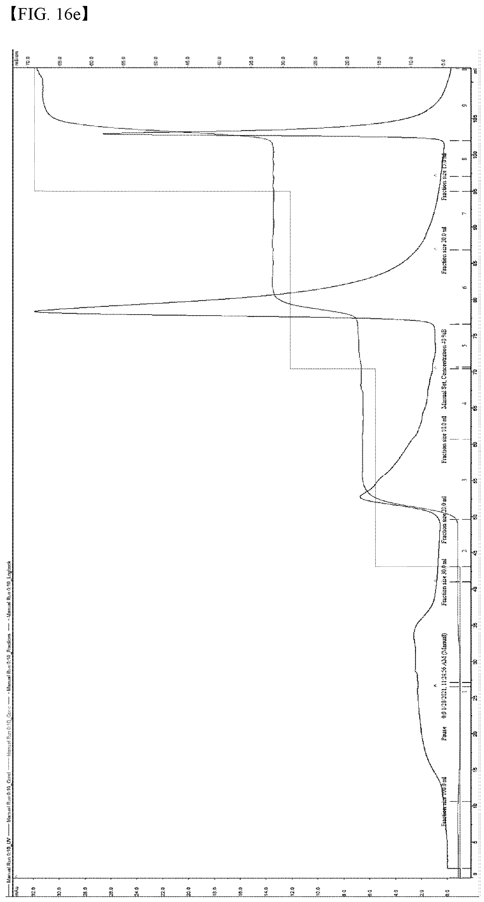

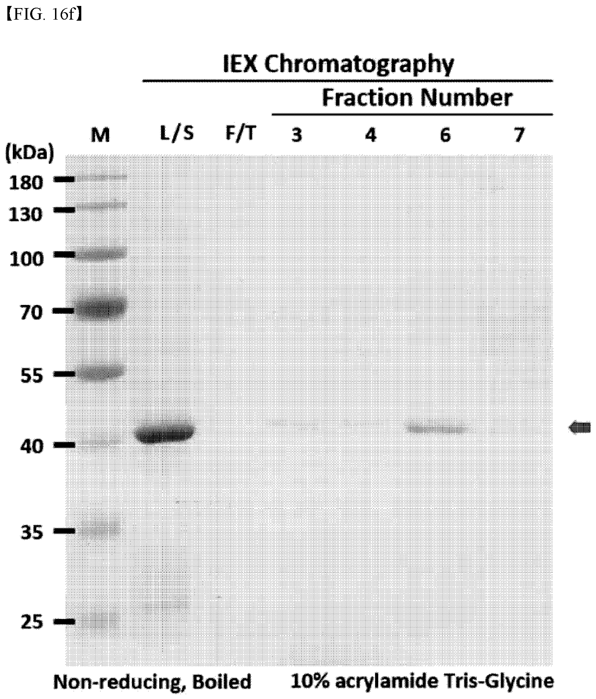

FIG. 14a is a mimetic diagram of an encapsulin protein-based virus-like particle expressing the EDIII antigen protein of dengue virus according to an exemplary embodiment of the present invention and a vaccine prepared thereby. FIG. 14b shows the structure of the expression vector corresponding to FIG. 14a, and it is an image of the vector structure of pET9a-MSAVKAA-RID-Linker1-TEV-Linker2-Encapsulin-Linker3-Dengue virus EDIII, which was inserted with additional sequences for the efficiency of MSAVKAA-RID and TEV Cleavage, which are expression level and water solubility enhancement fusion partners. The effects on the fusion expression of the fusion protein of the encapsulin protein and the target protein when RID, which is a fusion partner for enhancing expression level and water solubility, were confirmed through the results of FIGS. 15 and 16.
FIG. 15 is the result of confirming the effects on the expression level and the water-soluble expression of the fusion protein of encapsulin and the target protein according to the application of RID, which us a water-soluble enhancing fusion partner. In the case of a protein fused with EDIII (size 11.4 kDa), which is an antigen of dengue virus (DENV), to the C-terminus of encapsulin, the protein showed an insoluble expression pattern, but when it was expressed by the fusion of RID to the N-terminus, the result confirming that most of them were expressed in a water-soluble form was confirmed through SDS-PAGE.
FIG. 16a is a chromatogram result when RID-Encapsulin-Linker4-DENV EDIII protein (size: 53.1 kDa) expressed according to an exemplary embodiment of the present invention was purified through nickel affinity chromatography, and FIG. 16b is the result of confirming the separation pattern of RID-Encapsulin-Linker4-DENV EDIII protein by SDS-PAGE. FIG. 16c is a chromatogram result when the Encapsulin-Linker4-DENV EDIII protein fusion was purified by separating the N-terminal RID and the TEV cleavage site in nickel affinity chromatography, and FIG. 16d is an SDS-PAGE result confirming the purification pattern and purity of the fusion protein in the purification process ranging from TEV enzyme treatment and secondary nickel affinity chromatography of the Encapsulin-Linker4-DENV EDIII protein. FIG. 16e is a chromatogram result when the Encapsulin-Linker4-DENV EDIII protein fusion was purified through ion exchange resin chromatography in nickel affinity chromatography, and FIG. 16f is an SDS-PAGE result confirming the purification pattern and purity of the fusion protein.

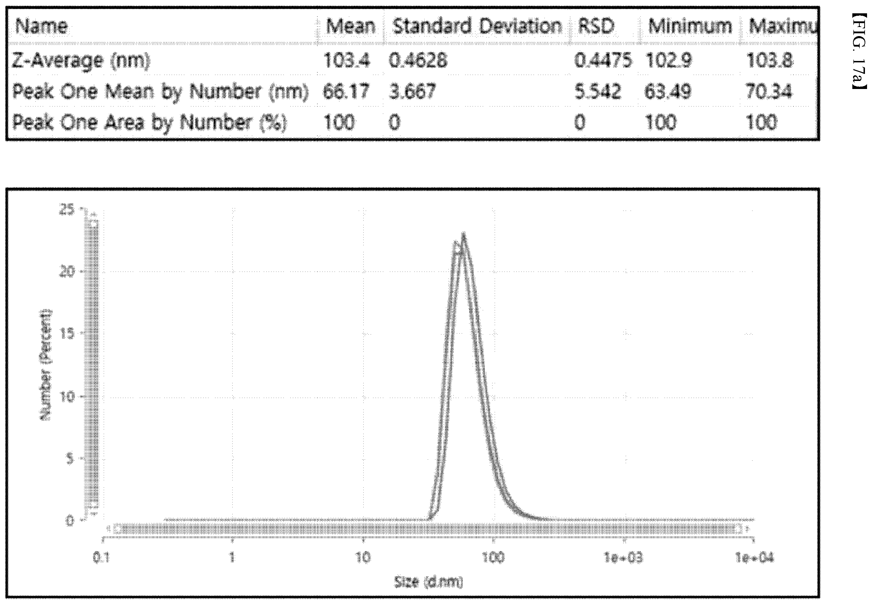

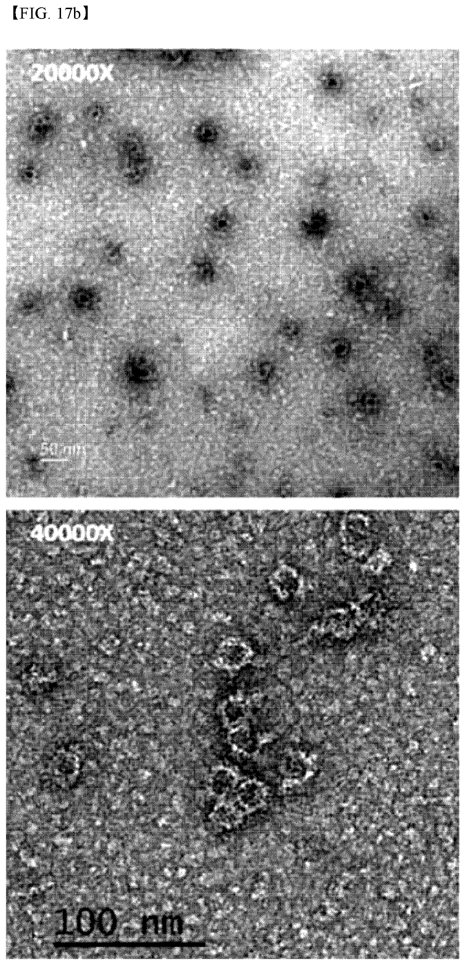

FIG. 17a shows the result of analysis through dynamic light scattering (DLS) to confirm the overall diameter distribution of the VLP of the purified encapsulin protein-target protein fusion protein. FIG. 17b shows the results of confirming the overall appearance of VLP of the encapsulin-based fusion protein with a target protein expressed on the surface through a purified transmission electron microscope (TEM).

### [Modes of the Invention]

Hereinafter, preferred exemplary embodiments of the present invention will be described in detail. The advantages and features of the present invention will be apparent with reference to the exemplary embodiments described below for achieving the same. However, the present invention is not limited to the exemplary embodiments to be disclosed below, but may be implemented in a variety of different forms. However, these exemplary embodiments are provided to make the disclosure of the present invention complete, and to fully inform the scope of the invention to those skilled in the art to which the present invention pertains, and the present invention is defined by the scope of the claims. The same reference numerals refer to the same constitutional elements throughout the specification.

Unless otherwise defined, all terms (including technical and scientific terms) used in the present specification may be used as meanings that can be commonly understood by those of ordinary skill in the art to which the present invention pertains. In addition, terms that are defined in a commonly used dictionary are not interpreted ideally or excessively unless explicitly defined specifically. The terms used in the present specification are for describing the exemplary embodiments and are not intended to limit the present invention. In the present specification, the singular form also includes the plural form unless specifically stated in the phrase.

The present invention provides a recombinant expression vector for vaccine production, including polynucleotides that encode an encapsulin protein and a target protein.

As used herein, the term "encapsulin protein" may be fused to a target protein and expressed together to enhance the expression efficiency of the protein. According to an exemplary embodiment of the present invention, the encapsulin protein includes the amino acid sequence of SEQ ID NO: 1 or a partial sequence thereof.

The encapsulin protein used in the present invention may be obtained from *Thermotoga maritima, Pyrococcus furiosus, Myxococcus xanthus* and the like, and most preferably from *Thermotoga maritima.* The encapsulin protein obtained from *Thermotoga maritima* is more advantageous for use as a vaccine because it can form small-sized particles.

About 60 encapsulin proteins of the present invention may be self-assembled to form particles having a diameter of 30 to 32 nm. This has the advantage of being able to maintain a stable form even with changes in pH and temperature. Therefore, various cargo proteins or substances may be loaded, and various antigens may be attached to the exposed surface domain (preferably C-terminal). The recombinant expression vector prepared according to an exemplary embodiment of the present invention may be expressed and self-assembled in various yeasts or cultured cells in addition to bacteria by using an encapsulin protein.

As used herein, the term "target protein" refers to a protein that a person skilled in the art wants to mass-produce, and it refers to a protein which can be expressed in a host cell by inserting a polynucleotide encoding the protein into a recombinant expression vector.

According to an exemplary embodiment of the present invention, the target protein includes a viral antigen protein, an antibody, a cell receptor, an enzyme, a structural protein, a serum and a cellular protein. According to a preferred exemplary embodiment of the present invention, the viral antigen protein includes any one of the gene sequences of SEQ ID NO: 2 and SEQ ID NO: 10 or a partial sequence thereof.

According to the present invention, the viral antigen protein corresponding to the target protein includes a rotavirus antigen protein and a cervical cancer virus antigen protein, but is not limited thereto. For example, it may be an antigen protein having a size of 10 to 300 amino acids, and preferably, a viral antigen protein having a size of 20 to 25 amino acids.

According to another exemplary embodiment of the present invention, the viral antigen protein corresponding to the target protein includes a coronavirus antigen protein and a dengue virus antigen protein, but is not limited thereto. For example, it may be an antigen protein having a size of 10 to 300 amino acids, and preferably, a viral antigen protein having a size of 20 to 223 amino acids.

In the present invention, the dengue virus may form four or more antigen proteins depending on the serotype, and for example, it is as represented by SEQ ID NO: 5 (Dengue virus Type 1 EDIII (KP406804.1)), SEQ ID NO: 6 (Dengue Virus Type 2 EDIII (EF654110.1)), SEQ ID NO:7 (Dengue Virus Type 3 EDIII (KP406805.1)) and SEQ ID NO:8 (Dengue Virus Type 4 EDIII (KP406806.1)).

As used herein, the term "expression vector" is a linear or circular DNA molecule composed of a fragment encoding a target protein which is operably linked to additional fragments that are provided for transcription of the expression vector. Such additional fragments include promoter and termination coding sequences. In addition, expression vectors include one or more origins of replication, one or more selectable markers and the like. Expression vectors are generally derived from plasmid or viral DNA, or contain elements of both. As used herein, the term "operably linked" refers to the arrangement of fragments in a promoter to act to initiate transcription and progress through the coding sequence to the termination code.

In the expression vector according to the present invention, the expression vector may be a plasmid, a viral vector, a phage particle or a genome insert. After the expression vector is transformed into a host cell, it may be cloned independently of the genome of the host cell or integrated into the genome of the host cell.

In addition, the present invention provides a recombinant expression vector for vaccine production, including polynucleotides that encode a target protein, an encapsulin protein and an RNA interacting domain (RID) protein. According to an exemplary embodiment of the present invention, the polynucleotide may further encode a "linker", and at least one linker protein may be positioned between the target protein, the encapsulin protein and the RID (RNA interacting domain) protein, respectively. The amino acid sequence of the linker may include at least one glycine or serine, and the linker may include the gene sequence of SEQ ID NO: 15, 16, 17, 18, 19 or 20. The position of each of the linkers is not limited, but a linker protein including the gene sequence of SEQ ID NO: 17 to SEQ ID NO: 20 is preferably positioned between the encapsulin protein and the antigen protein. In the present invention, "Linker 3" may be linker 3-1 represented by SEQ ID NO: 17, linker 3-1 represented by SEQ ID NO: 18, linker 3-3 represented by SEQ ID NO: 19 or linker 3-4 represented by SEQ ID NO: 20, and preferably, it may include the amino acid sequence "GGGSGGGSEAAAKGGGS."

As used herein, the terms "RID", "RNA interacting domain", "N terminal appended RNA binding domain of Lysyl tRNA synthetase" or "LysRS RNA interacting domain" refer to a unique N-terminal extension site involved in the interaction between the RNA of LysRS and other proteins.

According to an exemplary embodiment of the present invention, the RID protein may be used as a fusion partner to increase the expression level or water solubility of the vaccine.

According to a preferred exemplary embodiment of the present invention, the RID protein may include a domain for increasing the expression level of the vaccine (hereinafter, EE domain) and a domain for increasing water solubility (hereinafter, SE domain). For example, the EE domain may include the amino acid sequence of SEQ ID NO: 12 or a partial sequence thereof, and the SE domain may include the amino acid sequence of SEQ ID NO: 13 or SEQ ID NO: 14 or a partial sequence thereof.

In the recombinant expression vector for vaccine production of the present invention, the proteolytic enzyme may be TEV

According to a preferred exemplary embodiment of the present invention, the polynucleotide encoding the target protein may encode the TEV protein for RID cleavage.

In addition, the present invention provides a host cell which is transformed by the expression vector.

As used herein, the term "transformation" or "introduction" refers to introducing DNA into a host such that the DNA becomes replicable as an extrachromosomal factor or by chromosomal integration completion. The method for transforming the expression vector according to the present invention may include electroporation, the calcium phosphate (CaPO₄) method, the calcium chloride (CaCl₂) method, microinjection, the polyethylene glycol (PEG) method, the DEAE-dextran method, the cationic liposome method or the lithium acetate-DMSO method, but is not limited thereto.

In the host cell according to the present invention, the host cell is preferably a host cell having high DNA introduction efficiency and high DNA expression efficiency, and all microorganisms including prokaryotic and eukaryotic cells may be used. Preferably, the host cell may be *E. coli.*

In addition, the present invention provides a fusion protein including a target protein and an encapsulin protein, and the fusion protein may further include an RNA interacting domain (RID) protein.

As used herein, the term "fusion protein" refers to a protein in which another protein is linked or another amino acid sequence is added to the N-terminus or C-terminus of a target protein sequence. According to a preferred exemplary embodiment of the present invention, the target protein may be linked to the C-terminus of the encapsulin protein. In addition, the fusion protein in the present invention may be a vaccine produced by the recombinant expression vector of the present invention.

According to an exemplary embodiment of the present invention, a fusion protein may be formed by using the recombinant expression vector, and the fusion protein may be used as a vaccine as it is or through any optional additional processing.

The encapsulin protein according to the present invention may improve the expression efficiency of the target protein, and the fusion protein in which the encapsulin protein is bound to an RID, which is well known as a water-soluble enhancing protein, may be used for enhancing the expression efficiency of the target protein as well as for enhancing the water-solubility such that the encapsulin protein can be advantageously used in the production of fusion proteins.

According to another exemplary embodiment of the present invention, provided is a method for producing a vaccine, including the steps of a) producing a recombinant expression vector for vaccine production including polynucleotides that encode a target protein; an encapsulin protein; and an RID protein, b) producing a transformant by introducing the recombinant expression vector into a host cell and c) culturing the transformant to induce the expression of a recombinant fusion protein, and obtaining the same.

Hereinafter, the present invention will be described in more detail through examples.

These examples are only for illustrating the present invention, and it will be apparent to those of ordinary skill in the art that the scope of the present invention is not to be construed as being limited by these examples.

### [Example 1]

### Preparation of fusion protein including encapsulin

### <Example 1-1>

The encapsulin (*Thermotoga maritima* MSB8 (Sequence ID: NC_000853, Protein ID: WP_004080898.1)) gene sequence from *Thermotoga maritama* and having the protein structure of FIG. 1a was used for the high-efficiency production of a fusion protein including encapsulin. (SEQ ID NO: 1). Based on the gene sequence above, a gene sequence was prepared by synthesizing after codon optimization in *E. coli* (SEQ ID NO: 1) (the gene sequence of Example 1-1 was prepared by requesting BIONICS Co., Ltd. to synthesize the encapsulin gene). Afterwards, for the purpose of purifying encapsulin, a linker having the sequence as shown in SEQ ID NO: 17 was inserted at the C-terminus (amino acid sequence GGGSGGGSHHHHHHGGGS (SEQ ID NO: 17), (HHHHHH: amino acid corresponding to 6xHIS tag)). The complex thus prepared was named "Enc." The gene of the above sequence was inserted by using NdeI and BamHI, which are cleavage enzymes present in the MCS of the pET-9a (Novagene, 69431-3CN) (SEQ ID NO: 11) expression vector.

### <Example 1-2>

The expression vector constructed in Example 1-1 was transformed into HMS174 competent cells (Novagen (RecA mutation in a K-12 strain, Cat: 69453-3)). All transformed *E. coli* were cultured in 3 mL of LB medium containing 50 µg/mL kanamycin at 37°C at 250 rpm for 5 to 7 hours, then 1 mL was transferred to 10 mL, diluted 10 times and then further cultured. After about 2 to 4 hours, when O.D600 of 0.8 to 0.9 was reached, 0.4 mM IPTG (Biosesang, Cat#I1006, Lot#LB0C0340, cas#367-93-1) was added, and it was cultured for 17 to 19 hours at 16°C (up to 21 hours). For the culture medium, only the *E. coli* that was precipitated through centrifugation was obtained and stored frozen at -80°C. 0.3 mL of a lysis buffer solution (50 mM Tris-HCl (pH 7.5), 150 mM NaCl, 5% glycerol, 0.1% Triton X-100 and 10 mM imidazole) was added to the *E. coli* harvest corresponding to 3 mL of the culture medium, and after performing sonication, the whole lysate (T, Total lysate) was separated into a precipitate (P, Precipitant) and a supernatant (S, Soluble lysate) through centrifugation, and then, the separated protein was analyzed by SDS-PAGE.

FIG. 1 is a mimetic diagram of the recombinant expression vector for producing encapsulin protein according to an exemplary embodiment of the present invention and the expression results of a highly efficient water-soluble protein. FIG. 1a shows the protein structure of encapsulin predicted by using PDB, and FIG. 1b is a mimetic diagram showing the structure of the pET9a-Encapsulin recombinant expression vector. As shown in FIG. 1c, it was confirmed by SDS-PAGE whether the high-efficiency water-soluble expression of the encapsulin protein was performed by using the expression vector. The protein expressed according to Examples 1-1 and 1-2 was confirmed to be soluble in *E. coli* with a size of 32.1 kDa.

### <Example 1-3>

Cells obtained by culturing and inducing the expression of 500 mL of *E. coli* by using the culture method in the same manner as in Example 1-2 were resuspended with 75 mL of a lysis buffer [Buffer A [50 mM Tris-HCl (pH 7.5), 150 mM NaCl), 5% glycerol, 0.1% Triton X-100 and 10 mM imidazole]. Cells in the cell resuspension were disrupted by using an ultrasonic cell disrupter (sonication: pulse 3s/17s, 35% amplitude, 1 min 30 s, 4 times total). The lysed cells were centrifuged at 13,500 rpm at 4°C for 10 minutes, and the supernatant was collected and Ni+-affinity chromatography was performed by using AKTA (GE Healthcare). First, HisTrap HP (GE Healthcare Life Sciences, Little Chalfont, Buckinghamshire, UK, 5mL size) column, which is a cation-exchange resin column, was equilibrated by Buffer A (50 mM Tris-HCl (pH 7.5), 150 mM NaCl, 5% glycerol, 0.2 % Tween-20 and 10 mM imidazole), and then, the aqueous solution containing the water-soluble protein was loaded onto the equilibrated column at a flow rate of 1 mL/min. Purification of the encapsulin protein was performed by using Buffer A and Buffer B containing 1M imidazole (50 mM Tris-HCl (pH 7.5), 150 mM NaCl, 5% glycerol, 0.2% Tween-20 and 1M imidazole) in a form where imidazole was injected incrementally in a linear concentration gradient in the concentration range of 10 mM to 1M, and fractions of 2 mL each were obtained through this.

As shown in FIGS. 2a and 2b, after the separation pattern of the encapsulin protein of the elute was confirmed through SDS-PAGE, the eluate of the corresponding fraction was dialyzed against a dialysis buffer (50 mM Tris-HCl (pH 8.5), 10 mM NaCl, 0.2% Tween-20) at 9°C for 18 hours.

A biochemical analysis was performed to determine whether the encapsulin protein after the primary purification and dialysis formed VLP, and chromatography (ion exchange chromatography) was performed. Encapsulin proteins obtained by the dialysis were purified by ion exchange resin chromatography by using the FPLC system (AKTA, GE Healthcare). The column (HiTrap Q FF, 1 mL size (GE Healthcare Life Sciences, Little Chalfont, Buckinghamshire, UK)) was equilibrated with Buffer A (50 mM Tris-HCl (pH 7.5), 10 mM NaCl, 5% glycerol, 0.2% Tween-20) and loaded at a flow rate of 1 mL/min. The encapsulin protein was eluted by injecting NaCl having a linear concentration gradient of 10 mM to 1 M, and through Buffer Solution A and Buffer Solution B (50 mM Tris-HCl) (pH 7.5), 1M NaCl, 5% glycerol, 0.2% Tween-20), purification was performed through a linear concentration gradient, and fractions of 1 mL each were obtained.

A fusion protein in which other proteins derived from *E. coli* were purified through ion exchange resin chromatography by using a linear gradient (10 mM → 1M NaCl) was obtained (FIGS. 2c and 2d). The purified encapsulin protein was stored at 4°C in a state of containing a 20% glycerol ratio.

Additionally, purification using size exclusion chromatography (SEC) was performed. Analysis was performed by using the FPLC system (AKTAPurifier, GE Healthcare) (FIGS. 3a and 3b). The prepared sample was eluted by passing through a gel-filtration column (Superose^{™} 6 Increase 10/300 GL (GE Healthcare Life) Sciences)), which was equilibrated with a buffer solution (50 mM Tris-HCl (pH 7.5), 10 mM NaCl, 5% glycerol) under the same conditions as the dialysis solution. The elution pattern of size exclusion chromatography was measured at a wavelength of 280 nm and collected by using the Unicorn program (GE Healthcare Life Sciences).

As shown in FIG. 3b, the final purification of the encapsulin protein expressed in *E. coli* in a water-soluble and highly efficient manner was confirmed, and the concentration of the final purified protein was quantified by using BSA (Amresco, Solon, OH, USA).

### <Example 1-4>

In order to confirm the overall diameter distribution of the fusion protein (VLP) containing the purified encapsulin protein, analysis was performed through dynamic light scattering (DLS). Analysis of the complex was measured by placing 1 mL of a sample in a cuvette at a temperature of 16°C by using DLS (Particular Systems, Zetasizer Nano Family). Dynamic light scattering analysis of cells was confirmed by using the total intensity and mass for each area in the image. As shown in FIGS. 3c and 3d, it was confirmed that the diameter in the Hydrodynamic Radius of DLS appeared in a size similar to that of the wild-type encapsulin of 30 to 40 nm.

### <Example 1-5>

The appearance of the fusion protein (VLP) containing the purified encapsulin protein was observed with an electron microscope. The purified encapsulin VLP was first placed on a copper grid for 1 minute, then stained with 2% uranyl acetate for 1 minute, dried at room temperature for 10 minutes, and then photographed by using a transmission electron microscope (TEM, Transmission electron microscope (120 kV); Talos L120C, FEI, Czech). The above obtained results, as shown in FIG. 3e, confirmed that the purified encapsulin protein formed VLP. The diameter of the confirmed VLP was confirmed between 30 to 40 nm, which was similar to the diameter of the wild-type encapsulin.

### [Example 2]

### Preparation of encapsulin rotavirus antigen complex with N-terminal expression level and water solubility enhancement partners added for high efficiency and water solubility enhancement

### <Example 2-1>

VP8* (VP8 core region), which is a rotavirus antigen, was inserted to create an encapsulin-based virus-like particle composed of an encapsulin protein fusion protein with a target protein attached to the C-terminus. The VP8 core region (Human rotavirus A strain Wa G1P[8], (NCBI access number: FJ423116)) gene derived from rotavirus G1P[8] was used for the inserted rotavirus (SEQ ID NO: 2). VP8*, which is an antigen of rotavirus, is composed of 159 amino acids, and the molecular weight of the protein corresponds to about 18.3 kDa. Based on the protein sequence, a synthesized gene sequence was prepared after codon optimization in *E*. *coli* (SEQ ID NO: 2) (the encapsulin gene synthesis was requested at BIONICS Co., Ltd.).

In FIG. 4a, MSAVKAA (SEQ ID NO: 12), an RID peptide (SEQ ID NO: 13) and a mutant RID peptide with 50% or more similarity (SEQ ID NO: 14), which have the effects of increasing the expression level and increasing the water solubility, were fused at the N-terminus of the encapsulin protein to prepare a recombinant protein, and the water-soluble expression pattern was verified.

In addition, a peptide having the amino acid sequence of (GaSb)n (a≥1, b≥1, n≥1) was used as a linker of the optimized sequence by applying ENLYFQG/S, which is the sequence of the TEV cleavage site, and it was placed between the TEV cleavage sequence and the fusion protein sequence. A mimetic diagram of the fusion protein expression vector constructed in this example is shown in FIG. 4b.

The gene of the above sequence was constructed based on the pET-9a-Encapsulin vector, which is the recombinant complex protein expression vector constructed in Example 1 by using the pET-9a (Novagene, 69431-3CN) (SEQ ID NO: 11) expression vector. T7 RNA polymerase is expressed by IPTG, through which the RID sequence for increasing the water solubility and expression level was added to the N-terminus of the existing encapsulin complex protein vector sequence which was inserted into the MCS (Multiple Cloning Site) of the pET vector in which the Lac operon and T7 promoter present on the DE3 genome operate, and a linker sequence was added to enhance the TEV cleavage efficiency. The pET9a vector was cut out by using Nde1, KpnI, and BamHI cleavage enzymes, subcloned and reinserted. Among the cleavage enzyme sites present inside MCS, the RID and linker (Linker1) of this study, the cleavage enzyme site and the linker (Linker2) for increasing the cleavage efficiency of the TEV enzyme were inserted between the NdeI and KpnI sequences, and DNA sequences designed to have a protein in the form where encapsulin and the rotavirus antigen protein were fused were respectively inserted between the KpnI and BamHI cutting enzymes. In addition, each gene was linked to the pET vector and the inserted gene sequence through T4 DNA ligase, and it was confirmed through DNA sequence analysis that the bacterial clones generated by the ligation reaction of these DNAs had a codon-optimized nucleotide sequence inserted therein.

The sequence of the recombinant complex expression vector prepared in Example 2-1 is as follows.

ENC-Linker-VP8* [(SEQ ID NO: 1)-(SEQ ID NO: 17)-(SEQ ID NO: 2)]

:MEFLKRSFAPLTEKQWQEIDNRAREIFKTQLYGRKFVDVEGPYGWE
YAAHPLGEVEVLSDENEVVKWGLRKSLPLIELRATFTLDLWELDNLERGKPN
VDLSSLEETVRKVAEFEDEVIFRGCEKSGVKGLLSFEERKIECGSTPKDLLEAI
VRALSIFSKDGIEGPYTLVINTDRWINFLKEEAGHYPLEKRVEECLRGGKIITT
PRIEDALVVSERGGDFKLILGQDLSIGYEDREKDAVRLFITETFTFQVVNPEAL
ILLKF-
GGGSGGGSHHHHHHGGGSLDGPYQPTTFTPPTDYWILINSNTNGVVYESTN
NSDFWTAVIAVEPHVNPVDRQYNVFGENKQFNVRNDSDKWKFLEMFRGSSQ
NDFYNRRTLTSDTRLVGILKYGGRIWTFHGETPRATTDSSNTANLNGISITIHS
EFYIIPRSQESKCNEYINNGL

The prepared complex was named "ENC-VP8*."

MSAVKAA-RID-Linker1-TEV-Linker2-ENC-Linker3-VP8^{∗}[(SEQ ID NO: 12)-(SEQ ID NO: 14)-(SEQ ID NO: 15)-TEV-(SEQ ID NO: 16)-(SEQ ID NO: 1)-( SEQ ID NO: 17)-(SEQ ID NO: 2)]

:MSAVKAAAAVQAAEVKVDGSEPKLSANELAARLAAEAAVAEAEAA
QAELSEKQLSQATAAATNHTTDNGVGPEEESVGGGSGGGS-ENLYFQ-
GSGSGSMEFLKRSFAPLTEKQWQEIDNRAREIFKTQLYGRKFVDVEGPYGWE
YAAHPLGEVEVLSDENEVVKWGLRKSLPLIELRATFTLDLWELDNLERGKPN
VDLSSLEETVRKVAEFEDEVIFRGCEKSGVKGLLSFEERKIECGSTPKDLLEAI

VRALSIFSKDGIEGPYTLVINTDRWINFLKEEAGHYPLEKRVEECLRGGKIITT

PRIEDALVVSERGGDFKLILGQDLSIGYEDREKDAVRLFITETFTFQVVNPEAL

ILLKF-

GGGSGGGSHHHHHHGGGSLDGPYQPTTFTPPTDYWILINSNTNGVVYESTN

NSDFWTAVIAVEPHVNPVDRQYNVFGENKQFNVRNDSDKWKFLEMFRGSSQ

NDFYNRRTLTSDTRLVGILKYGGRIWTFHGETPRATTDSSNTANLNGISITIHS

EFYIIPRSQESKCNEYINNGLL

The prepared complex was named "RID-ENC-VP8*."

### <Example 2-2>

The expression vector constructed in Example 2-1 was transformed into HMS174 competent cells (Novagen (RecA mutation in a K-12 strain, Cat: 69453-3)). All transformed *E. coli* were cultured in 3 mL of LB medium containing 50 µg/mL kanamycin at 37°C at 250 rpm for 5 to 7 hours, then 1 mL was transferred to 10 mL, diluted 10 times and then further cultured. After about 2 to 4 hours, when O.D600 of 0.8 to 0.9 was reached, 0.4 mM IPTG (Biosesang, Cat#I1006, Lot#LB0C0340, cas#367-93-1) was added, and it was cultured for 17 to 19 hours at 16°C (up to 21 hours). For the culture medium, only the *E. coli* precipitated through centrifugation was obtained and stored frozen at -80°C. 0.3 mL of a lysis buffer solution (50 mM Tris-HCl (pH 7.5), 150 mM NaCl, 5% glycerol, 0.1% Triton X-100 and 10 mM imidazole) was added to the *E. coli* harvest corresponding to 3 mL of the culture medium, and after sonicating, the whole lysate (T, Total lysate) was separated into a precipitate (P, Precipitant) and a supernatant (S, Soluble lysate) through centrifugation, and then analyzed by SDS-PAGE, and the results are shown in FIG. 4c. It was confirmed that the recombinant encapsulin protein constructed in Example 2-1 was expressed in a high-efficiency water soluble manner in *E. coli.* It was confirmed that Enc-VP8* and RID-ENC-VP8* were also expressed at appropriate positions corresponding to their respective sizes. However, the protein in which the rotavirus antigen was fused to the encapsulin had a size of 50.3 kDa, and when it was expressed by the fusion of a peptide or protein having a certain size or more to the C-terminus, it was confirmed that the water solubility of the fusion protein was significantly reduced. Therefore, in order to prepare a VLP complex having a foreign antigen exposed on the surface through fusion expression of the foreign antigen protein to the C-terminus of encapsulin derived from *Thermotoga maritama,* first, a water-soluble expression marker (Soluble Expression Tag), which significantly increases the high expression and water solubility of the fusion protein, is essentially required. In the present invention, an RID (RNA Interaction Domain), which significantly increases the expression level and water solubility, was fused to the N-terminus of encapsulin as a water-soluble expression marker, and the high-efficiency water-soluble expression of a fusion protein, in which an external antigen having a large molecular weight (about 18 kDa or more) was fused to the C-terminus thereof, was attempted. As a result, as shown in FIG. 4c, when a single protein of encapsulin was expressed, the protein had high water solubility, but when the antigen protein was fused to the C-terminus of the encapsulin, it was confirmed that a highly water-soluble protein was obtained only when the water-soluble marker expression factor of this experiment was added to the N-terminus and expressed. In addition, when a linker sequence having an appropriate length and sequence was added after the enzymatic cleavage sequence, the TEV enzyme cleavage efficiency was higher than 90%, making it possible to purify the final protein in high yield in the subsequent purification process.

### <Example 2-3>

Cells obtained by culturing and inducing the expression of 500 mL of *E. coli* by using the same culture method as in Example 2-2, in which the high-efficiency water-soluble expression was confirmed, were resuspended with 75 mL of a lysis buffer solution [Buffer A [50 mM Tris-HCl] (pH 7.5), 150 mM NaCl, 5% glycerol, 0.1% Triton X-100 and 10 mM imidazole]. Cells in the cell resuspension were disrupted by using an ultrasonic cell disrupter (sonication: pulse 3s/17s, 35% amplitude, 1 min 30 s, 4 times total). The lysed cells were centrifuged at 13,500 rpm at 4°C for 10 minutes, and the supernatant was collected and Ni+-affinity chromatography was performed by using AKTA (GE Healthcare). First, the HisTrap HP (GE Healthcare Life Sciences, Little Chalfont, Buckinghamshire, UK, 5mL size) column, which is a cation-exchange resin column, was equilibrated by Buffer A (50 mM Tris-HCl (pH 7.5), 150 mM NaCl, 5% glycerol, 0.2 % Tween-20 and 10 mM imidazole), and then, the aqueous solution containing the water-soluble protein was loaded onto the equilibrated column at a flow rate of 1 ml/min. Purification of the RIDEncapsulin-VP8* protein was performed by using Buffer A and Buffer B containing 1M imidazole (50 mM Tris-HCl (pH 7.5), 150 mM NaCl, 5% glycerol, 0.2% Tween-20 and 1M imidazole) in a form where imidazole was injected incrementally in a linear concentration gradient in the concentration range of 10 mM to 1M, and fractions of 2 mL each were obtained through this.

After confirming the separation pattern of the target protein for the eluate through SDS-PAGE, the eluate of the corresponding fraction was dialyzed with Dialysis Buffer ([50 mM Tris-HCl (pH 8.5), 10 mM NaCl, 0.2% Tween-20]) at 9°C for 12 to 16 hours. In this case, after dialyzing first for 4 hours, the TEV cleave enzyme was used to cleave between the fusion protein in which the N-terminal fusion partner RID, the encapsulin protein and the rotavirus antigen were fused for the remaining 8 to 12 hours.

A biochemical analysis was performed to determine whether the ENC-VP8* protein after IMAC and dialysis formed VLP, and for subsequent purification, ion exchange chromatography was performed by using the AKTA FPLC system (GE Healthcare). Encapsulin proteins obtained by the dialysis were purified by ion exchange resin chromatography by using the FPLC system (AKTA, GE Healthcare). The column (HiTrap Q FF, 1 mL size (GE Healthcare Life Sciences, Little Chalfont, Buckinghamshire, UK)) was equilibrated with Buffer A (50 mM Tris-HCl (pH 7.5), 10 mM NaCl, 5% glycerol, 0.2% Tween-20) and loaded at a flow rate of 1 mL/min. ENC-VP8* protein was eluted by injecting NaCl having a linear concentration gradient of 10 mM to 1 M, and through Buffer Solution A and Buffer Solution B (50 mM Tris-HCl (pH 7.5), 1M NaCl, 5% glycerol, 0.2% Tween-20), purification was performed through a linear concentration gradient, and fractions of 1 ml each were obtained. Through this, a fusion protein was obtained in which other proteins derived from *E. coli* were purified (FIG. 4d). The purified ENC-VP8* protein was stored at 4°C in a state of containing a 20% glycerol ratio, and the concentration of the finally purified protein was quantified by using BSA (Amresco, Solon, OH, USA).

### <Example 2-4>

In order to confirm the overall diameter distribution of the fusion protein (VLP) composed of the ENC-VP8* fusion protein of Example 2-3, analysis was performed through dynamic light scattering (DLS). Analysis of the complex was measured by placing 1 mL of a sample in a cuvette under a temperature condition of 16°C by using DLS (Particular Systems, Zetasizer Nano Family). Dynamic light scattering analysis of cells was confirmed by using the total intensity and mass for each area in the image. As shown in FIG. 5a, it was confirmed to have a distribution of particle sizes with a diameter of 30 to 40 nm in the Hydrodynamic Radius of DLS.

### <Example 2-5>

The appearance of virus-like particles (VLPs) containing the purified encapsulin protein was observed with an electron microscope. The purified encapsulin VLP was first placed on a copper grid for 1 minute, then stained with 2% uranyl acetate for 1 minute, dried at room temperature for 10 minutes, and then photographed by using a transmission electron microscope (TEM, Transmission electron microscope (120 kV)); Talos L120C, FEI, Czech). As shown in FIG. 5b, it was confirmed that the purified ENC-VP8* protein formed VLP. The diameter of the confirmed VLP appeared to be between 30 and 40 nm, which is the diameter of the existing encapsulin protein, and it was confirmed that a spherical VLP was formed even in the form of a fusion protein in which the antigen protein was fused to the C-terminus.

### <Example 2-6>

In order to measure the antigen-specific antibody inducibility, the finally purified ENC-VP8* fusion VLP protein was quantified by using BSA (Amresco, Solon, OH, USA), and then diluted to an appropriate concentration with a buffer containing 20% glycerol. Antigen and adjuvant (Alhydrogel^{®}, Invitrogen Cat# vac-alu-250) were mixed vigorously for more than 5 minutes by using a syringe at a 1:1 ratio, and then 100 µL was inoculated in a 4-week-old BALB/C mouse group (6 mice/group) via intramuscular injection. Vaccine was inoculated and immunized 3 times with ENC-VP8* VLP 5 µg or 10 µg at 2 week intervals, respectively, and serum was obtained through orbital blood sampling one day before vaccination. In order to measure the target protein-specific antibody in the serum obtained from each animal experimental group, the enzyme-linked immunoprecipitation assay (ELISA (enzyme-linked immunosorbent assay)) was performed. 100 µL of the rotavirus VP8 antigen protein (P2-VP8) used as a control was placed into a 96-well plate (Nunc MaxiSorp flat-bottom (Thermo Scientific)) at a concentration of 0.5 µg/mL per well, and after coating at 4°C for 18 hours, 200 µL of 3% BSA solution was added, and blocking was performed at 37°C for 2 hours. After washing the plate using PBST, the immune serum was diluted (1: 1,000 dilution ratio) to add 100 µL per well, and it was reacted in the same way at 37°C for 1 hour and 30 minutes. After washing the plate using PBST, an antibody against goat-derived mouse IgG (Southern Biotech 1030-05, Goat-anti mouse IgG HRP) was treated at 37°C for 1 hour to detect the antibody in the serum bound to P2-VP8*. After color development at room temperature for 10 minutes using TMB (Sigma T0440) solution, the color development reaction was stopped with 0.5M sulfuric acid solution, and absorbance was measured at a wavelength of 450 nm. As shown in FIG. 6b, when the VP8-specific antibody titer of ENC-VP8* present in the primary, secondary and tertiary blood samples was measured, respectively, it was confirmed that high levels of VP8-specific antibodies were formed in 10 µg of Encapsulin _VP8_High as well as in 5 µg of Encapsulin_VP8_Low group. Further, in the purified peptide protein of the VP8-specific antibody titer control, the antibody was confirmed from the tertiary 2nd Boost sera pool, whereas in Encapsulin _VP8_High and Encapsulin _VP8_Low, the formation of VP8-specific antibody was confirmed from the Boost sera pool, which is the secondary blood sample.

After Encapsulin-VP8* VLP inoculation, the neutralizing antibody titer of rotavirus (Human Wa strain) present in the blood was measured by the plaque-reduction neutralization test (PRNT) method. The virus was reacted with the serum of the inoculated animals diluted step by step at 37°C, followed by performing a plaque assay, and the PRNT titer based on the dilution factor showing a 50% reduction in viral plaque was determined compared to the PBS control group. After adding 100 ng of rotavirus VP8 antigen protein (P2-VP8) or 100 pfu of human rotavirus Wa Strain used as controls to a 96-well plate (Nunc MaxiSorp flat-bottom (Thermo Scientific^{™})) and coating at 4 degrees for 18 hours, 200 µL of 3% BSA solution was added, and it was blocked at 37°C for 2 hours. After washing the plate using PBST, 100 µL of an immune serum dilution (1:1000) was added per well, and the plate was reacted at 37°C for 1 hour and 30 minutes. After washing the plate using PBST, goat-derived anti-mouse IgG (Southern Biotech 1030-05, Goat-anti mouse IgG HRP) was treated at 37°C for 1 hour to detect the antibody in the serum bound to the antigen protein. After color development at room temperature for 10 minutes using TMB (Sigma T0440) solution, the color development reaction was stopped with 0.5M sulfuric acid solution, and absorbance was measured at a wavelength of 450 nm. As shown in FIG. 6c, the rotavirus-specific antibody titer present in the sample, in which blood was collected after inoculation of the control group P2-VP8* protein antigen three times, showed a significantly lower level, whereas the VP8-specific antibody titer present in the sample, in which blood was collected after inoculation of ENC-VP8* three times, showed a significantly high level. In particular, it was confirmed that a high level of VP8-specific antibody was formed not only in 10 µg of Encapsulin_VP8_High but also in 5 µg of Encapsulin _VP8_Low group, and it can be seen that the same results were obtained even when the specific antibody titer for human rotavirus was measured.

In addition, 100 pfu of human rotavirus Wa Strain was placed in a 96-well plate (Nunc MaxiSorp flat-bottom (Thermo Scientific^{™})) to confirm the continuity of immunity after the formation of VP8-specific antibody, and after coating at 4°C for 18 hours, 200 µL of 3% BSA solution was added, and it was blocked by reacting at 37°C for 2 hours. After washing the plate using PBST, 100 µL of an immune serum dilution (1: 1,000) was added per well, and the plate was reacted at 37°C for 1 hour and 30 minutes. After washing the plate using PBST, goat-derived anti-mouse IgG (Southern Biotech 1030-05, Goat-anti mouse IgGHRP) was treated at 37°C for 1 hour to detect the antibody in the serum bound to the antigen protein. After color development at room temperature for 10 minutes using TMB (Sigma T0440) solution, the color development reaction was stopped with 0.5M sulfuric acid solution, and absorbance was measured at a wavelength of 450 nm. As shown in FIG. 6d, the rotavirus-specific antibody titer present in the blood serum was measured 2 months after inoculation with the control group P2-VP8* protein antigen or ENC-VP8* three times. Compared to the serum inoculated with the control P2-VP8* protein antigen, it was confirmed that the antibody titer induced at a very high level in the serum of the animals inoculated with ENC-VP8* was still maintained to be high after 2 months.

In addition, HBGA binding assay was performed to confirm whether ENC-VP8 binds to HBGA. As shown in FIG. 6e, in a 96-well plate (High Binding Capacity (HBC) NeutrAvidin plates (Thermo Scientific^{™})) coated with streptavidin, 100 µL of HBGA bound with biotin was placed in each well at a concentration of 10 µg/mL, and it was reacted at room temperatures for 5 hours. After washing the plate using PBST, the ENC-VP8 protein was diluted in PBS, placed by 100 µL per well and reacted at 4°C for 18 hours. After washing the plate using PBST, mouse serum inoculated with ENC-VP8 was diluted at a ratio of 1: 1,000, placed by 100 µL per well, and reacted at room temperature for 1 hour. After washing with PBST, 5 types of antibodies (H (type 2)-PAA-biotin (01-034), Lea-PAA-biotin (01-035), Led (H type1)-PAA-biotin (01-037), Blood type A (tri)-PAA-biotin (01-032), Blood type B(tri)-PAA-biotin (01-033); (Glycotech)) against goat-derived mouse IgG (Goat-anti mouse IgG HRP) were treated at room temperature for 1 hour. After color development at room temperature using the TMB solution for 10 minutes, the color development reaction was stopped with 0.5 M sulfuric acid solution, and absorbance was measured at a wavelength of 450 nm. As shown in FIG. 6d, binding between HBGA and VP8 antigen was confirmed by using primary sera obtained from the group injected with ENC-VP8*, and as a result, it was confirmed that the ENC-VP8* VLP fusion protein binds to Led (H type1) carbohydrate.

### <Example 2-7>

In order to measure the degree to which immune serum inhibits the binding between ENC-VP8* antigen and receptor, HBGA blocking assay was performed (FIG. 6g). In a 96-well plate coated with streptavidin, 100 µL of HBGA bound with biotin was placed per well at a concentration of 10 µg/mL, and it was reacted at room temperature for 5 hours. ENC-VP8* was diluted to a concentration of 1 µg/mL and then reacted with immune serum at a ratio of 1:1 at 37°C for 3 hours. Afterwards, 100 µL of serum and the ENC-VP8* reaction solution were added per well to the plate reacted with HBGA, which was washed with PBST, and it was reacted at 4°C for 18 hours. After washing the plate using PBST, mouse serum inoculated with ENC-VP8* was diluted at a ratio of 1: 1,000, 100 µL thereof was placed per well, and it was reacted at room temperature for 1 hour. After washing with PBST, an antibody against goat-derived mouse IgG (Goat-anti mouse IgG HRP) was treated at room temperature for 1 hour. Afterwards, the color was developed using TMB solution at room temperature for 10 minutes, the color development reaction was stopped with 0.5 M sulfuric acid solution, and the absorbance was measured at a wavelength of 450 nm. As shown in FIG. 6h, after coating Led (H type 1) which confirmed the binding of ENC-VP8 VLP antigen protein in the HBGA Binding Assay on a plate, Encapsulin _VP8 VLP was reacted with immune sera (primary, boost, 2^{nd} boost) which was diluted to various concentrations, respectively, and the results of measuring whether it bound to Led were confirmed by ELISA.

FIG. 6i is a numerical representation of the HBGA blocking efficacy of each immune serum obtained in the above example. Each treatment group was a low-dose control group (P2-VP8_Low), a high-dose control group (P2-VP8_High), a low-dose test group (Encapsulin-VP8_Low) and a high-dose test group (Encapsulin-VP8_High), and after obtaining serum up to the tertiary inoculation of a total of 4 treatment groups, the HBGA binding inhibitory ability of each serum was measured. After performing the HBGA blocking assay of each immune serum, the dilution factor (BT50) showing a binding inhibitory effect of 50% compared to PBS was calculated.

In the case of the ENC-VP8 antigen fusion VLP protein of this example, the inhibitory ability of immune serum was confirmed from primary sera, which was the first inoculation, and as the inoculation was repeated up to the tertiary inoculation, the inhibitory ability of the immune serum increased significantly compared to the control groups. On the other hand, in the P2 VP8 group, which was a control group, immune serum inhibited antigen-receptor binding from the 2nd immune sera after the second vaccination, and it was confirmed that the protective effect was lower than that of the Enc_VP8 group even after the tertiary vaccination.

Through the results of Example 2, when the VLP in which Encapsulin and the target protein according to the present invention are fused, it was confirmed that a much higher level of the target protein-specific antibody was induced earlier than when only the target protein was administered, and the performance of inhibiting the binding between the target protein and its receptor was excellent.

### [Example 3]

### Preparation of encapsulin human papilloma antigen complex with N-terminal peptide added for high efficiency and water solubility enhancement

### <Example 3-1>

In order to examine whether it is possible to form a VLP composed of a virus-like particle based on the fusion protein encapsulin when a protein smaller than the rotavirus VP8* antigen of about 18.3 kDa in size consisting of 159 amino acids is inserted at the C-terminus, the human papillomavirus (HPV) antigen was used. E7 LP, which is a human papillomavirus antigen, was inserted as an encapsulin protein, and the E7 core region (E7 protein, partial [Human papillomavirus type 16] Sequence ID: ABL96584.1) gene derived from human papillomavirus HPV 16 was used (SEQ ID NO: 9). E7 LP (Long peptide), which is an antigen of human papillomavirus, consists of 35 amino acids and has a size of about 4 kDa. Based on the protein sequence, a gene sequence synthesized after codon optimization in *E. coli* was prepared (SEQ ID NO: 9) (the encapsulin gene synthesis was requested at BIONICS Co., Ltd.).

Human papillomavirus E7LP was inserted at the C-terminus of the encapsulin protein. In addition, as shown in FIG. 8a, the effect on the expression of the fusion protein was confirmed by applying RID, which is a fusion partner to enhance the expression level and water solubility, to the N-terminus of the encapsulin protein to enhance high efficiency and water solubility. Accordingly, MSAVKAA (SEQ ID NO: 12), an RID peptide (SEQ ID NO: 13) and a mutant RID peptide with 50% or more similarity (SEQ ID NO: 14), having the effect of increasing the expression level and water solubility, were fused to prepare a recombinant protein, and the water-soluble expression pattern was verified.

In addition, a peptide having the amino acid sequence of (GaSb)n (a≥1, b≥1, n≥1) was used as a linker of the optimized sequence by applying ENLYFQG/S, which is the sequence of the TEV cleavage site, and it was placed between the TEV cleavage sequence and the fusion protein sequence. A mimetic diagram of the fusion protein expression vector constructed in this example is shown in FIG. 7b.

The gene of the above sequence was constructed based on the pET9a-Encapsulin-VP8* vector and the pET9a-MSAVKAA-RID-Linker-Enc-Linker-VP8* vector, which are the recombinant complex protein expression vectors constructed in Example 2 by using the pET-9a (Novagene, 69431-3CN) (SEQ ID NO: 11) expression vector, and the rotavirus antigen protein at the C-terminus of Enc was replaced with a human papillomavirus antigen by using NcoI and BamHI cleavage enzymes and inserted.

The sequence of the recombinant complex expression vector prepared in Example 3-1 is as follows.
ENC-Linker-E7 LP[(SEQ ID NO: 1)-(SEQ ID NO: 17)-(SEQ ID NO: 9)]

MEFLKRSFAPLTEKQWQEIDNRAREIFKTQLYGRKFVDVEGPYGWEYAAHPL
GEVEVLSDENEVVKWGLRKSLPLIELRATFTLDLWELDNLERGKPNVDLSSL
EETVRKVAEFEDEVIFRGCEKSGVKGLLSFEERKIECGSTPKDLLEAIVRALSI

FSKDGIEGPYTLVINTDRWINFLKEEAGHYPLEKRVEECLRGGKIITTPRIEDA
LVVSERGGDFKLILGQDLSIGYEDREKDAVRLFITETFTFQVVNPEALILLKF-
GGGSGGGSHHHHHHGGGS-
GQAEPDRAHYNIVTFCCKCDSTLRLCVQSTHVDIR

### [Encapsulin-Linker-HPV E7] [(SEQ ID NO: 1)- (SEQ ID NO: 17)-(SEQ ID NO: 9)]

The prepared complex was named "Enc-E7 LP."
MSA VKAA - RID-Linker 1-TEV-Linker2-ENC-Linker-E7 LP
[(SEQ ID NO: 12)-(SEQ ID NO: 13)-(SEQ ID NO: 15)-TEV-(SEQ ID NO: 16)-(SEQ ID NO: 1)-(SEQ ID NO: 17)-(SEQ ID NO: 9)]

:MSAVKAA-
AAVQAAEVKVDGSEPKLSKNELKRRLKAEKKVAEKEAKQKELSEKQLSQAT
AAATNHTTDNGVGPEEESV-ENLYFQ-
GSGSGSMEFLKRSFAPLTEKQWQEIDNRAREIFKTQLYGRKFVDVEGPYGWE
YAAHPLGEVEVLSDENEVVKWGLRKSLPLIELRATFTLDLWELDNLERGKPN
VDLSSLEETVRKVAEFEDEVIFRGCEKSGVKGLLSFEERKIECGSTPKDLLEAI
VRALSIFSKDGIEGPYTLVINTDRWINFLKEEAGHYPLEKRVEECLRGGKIITT
PRIEDALVVSERGGDFKLILGQDLSIGYEDREKDAVRLFITETFTFQVVNPEAL
ILLKF-GGGSGGGSHHHHHHGGGS-
GQAEPDRAHYNIVTFCCKCDSTLRLCVQSTHVDIR

The prepared complex was named "RID-ENC-E7 LP" (LP stands for Long Peptide).

### <Example 3-2>

The expression vector constructed in Example 3-1 was transformed into HMS174 competent cells (Novagen (RecA mutation in a K-12 strain, Cat: 69453-3)). All transformed *E. coli* were cultured in 3 mL of LB medium containing 50 µg/mL kanamycin at 37°C at 250 rpm for 5 to 7 hours, and then 1 mL was transferred to 10 mL, diluted 10 times and then further cultured. After about 2 to 4 hours, when O.D600 reached 0.8 to 0.9, 0.4 mM IPTG (Biosesang, Cat#I1006, Lot#LB0C0340, cas#367-93-1) was added, and it was cultured for 17 to 19 hours at 16°C (up to 21 hours). For the culture medium, only the *E. coli* precipitated through centrifugation was obtained and stored frozen at -80°C. 0.3 mL of a lysis buffer solution (50 mM Tris-HCl (pH 7.5), 150 mM NaCl, 5% glycerol, 0.1% Triton X-100 and 10 mM imidazole) was added to the *E. coli* harvest corresponding to 3 mL of culture medium, and after sonicating, the whole lysate (T, Total lysate) was separated into a precipitate (P, Precipitant) and a supernatant (S, Soluble lysate) through centrifugation, and then it was analyzed by SDS-PAGE. As a result, as shown in FIGS. 7c and 7d, as a result of confirming by expressing ENC-E7 LP and RID-ENC-E7 LP corresponding to each size, respectively, 36.1 kDa and 45.7 kDa proteins were expressed at appropriate positions. However, it was confirmed that the solubility of the fusion protein was significantly reduced even when the human papillomavirus of 35 amino acids and 4 kDa smaller than the 18 kDa rotavirus was fused to the C-terminus of the encapsulin, and through this, it was confirmed that RID, which is a high-efficiency and water-soluble enhancement fusion partner, is required to be expressed in *E. coli* as all of encapsulin complexes, ranging from short-sequenced peptides, small-sized proteins to large-sized proteins. Therefore, similar to the results of Example 2, in order to prepare a VLP complex having a foreign antigen exposed on the surface through fusion expression of the foreign antigen protein to the C-terminus of the encapsulin, it was reconfirmed that the water-soluble expression marker of the present invention is essentially required, and it demonstrated the necessity for the an RID (RNA Interaction Domain) and water-soluble expression marker, which significantly increase the high expression level and water solubility in the present invention, in the *E. coli* expression system.

### <Example 3-3>

The RID-ENC-E7 LP complex protein prepared by using the same culture method as in Example 3-2, whose water solubility was confirmed, was purified through nickel (Ni) affinity chromatography. Cells obtained by culturing and inducing the expression of 500 mL of *E. coli* using the same culture method were resuspended with 75 mL of a lysis buffer solution [Buffer A [50 mM Tris-HCl (pH 7.5), 150 mM NaCl, 5% glycerol, 0.1% Triton X-100 and 10 mM imidazole]. Cells in the cell resuspension were disrupted by using an ultrasonic cell disrupter (sonication: pulse 3s/17s, 35% amplitude, 1 min 30 s, 4 times total). The lysed cells were centrifuged at 13,500 rpm at 4°C for 10 minutes, and the supernatant was collected and Ni+-affinity chromatography was performed using AKTA (GE Healthcare). First, the HisTrap HP (GE Healthcare Life Sciences, Little Chalfont, Buckinghamshire, UK, 5mL size) column, which is a cation-exchange resin column, was equilibrated with Buffer A (50 mM Tris-HCl (pH 7.5), 150 mM NaCl, 5% glycerol, 0.2 % Tween-20 and 10 mM imidazole), and then the aqueous solution containing the water-soluble protein was loaded onto the equilibrated column at a flow rate of 1 mL/min. Purification of RID-Encapsulin-VP8* protein was performed by using Buffer A and Buffer B containing 1M imidazole (50 mM Tris-HCl (pH 7.5), 150 mM NaCl, 5% glycerol, 0.2% Tween-20 and 1M imidazole) in the form where imidazole was incrementally injected in a linear concentration gradient in a concentration range of 10 mM to 1 M, and fractions of 2 mL each were obtained through this.

As shown in FIGS. 8a and 8b, after confirming the separation pattern of the target protein through SDS-PAGE for the eluate, the eluate of the corresponding fraction was treated with Dialysis buffer ([50 mM Tris-HCl (pH 8.5), 10 mM NaCl, 0.2% Tween-20]) at 9°C for 12 to 16 hours. In this case, after dialyzing first for 4 hours, the fusion protein, in which the N-terminal fusion partner RID and the encapsulin protein and the human papillomavirus antigen were fused, was cleaved by using the TEV cleaving enzyme for the remaining 8 to 12 hours.

A biochemical analysis was performed to determine whether the ENC-E7 LP protein after IMAC and Dialysis formed VLP, and for subsequent purification, ion exchange chromatography was performed using the AKTA FPLC system (GE Healthcare). Encapsulin proteins obtained by the above dialysis were purified by ion exchange resin chromatography by using the FPLC system (AKTA, GE Healthcare). The column (HiTrap Q FF, 1 mL size (GE Healthcare Life Sciences, Little Chalfont, Buckinghamshire, UK)) was equilibriated with Buffer A (50 mM Tris-HCl (pH 7.5), 10 mM NaCl, 5% glycerol, 0.2% Tween-20) and loaded at a flow rate of 1 mL/min. The Encapsulin-E7 LP protein was eluted by injection of NaCl having a linear concentration gradient of 10 mM to 1 M, and purification through a linear concentration gradient was performed through Buffer Solution A and Buffer Solution B (50 mM Tris-HCl (pH 7.5), 1M NaCl, 5% glycerol, 0.2% Tween-20), and fractions of 1 mL each were obtained. Through this, the fusion protein was obtained, in which other proteins derived from *E. coli* (FIG. 8d). The purified ENCE7 LP protein was stored at 4°C with a 20% glycerol ratio, and the concentration of the finally purified protein was quantified by using BSA (Amresco, Solon, OH, USA). As shown in FIG. 9a, the purification pattern and purity of the fusion protein were confirmed in the purification process from the protein expression of the RID and additional markers for water solubility according to the present invention to TEV enzyme treatment and ion exchange resin chromatography, and it was confirmed that even in the case of an antigen of human papillomavirus, it is essential for stably acquiring the protein.

### <Example 3-4>

In order to confirm the overall diameter of the purified virus-like particles (VLPs) of the ENC-E7 LP complex of Example 3-3, analysis was performed through dynamic light scattering (DLS). Analysis of the complex was measured by placing 1 mL of the sample in a cuvette at a temperature of 16°C by using DLS (Particular Systems, Zetasizer Nano Family). Dynamic light scattering analysis of cells was confirmed by using the total intensity and mass for each area in the image. As shown in FIG. 9b, it was confirmed that the diameter in the Hydrodynamic Radius of DLS had a size distribution similar to that of 30 to 40 nm in which the wild-type encapsulin appeared.

### <Example 3-5>

The appearance of virus-like particles (VLPs) containing the purified encapsulin protein was observed with an electron microscope. The purified Encapsulin VLP was first placed on a copper grid for 1 minute, then stained with 2% uranyl acetate for 1 minute, dried at room temperature for 10 minutes, and then photographed by using a transmission electron microscope (TEM, Transmission electron microscope (120 kV)); Talos L120C, FEI, Czech). As shown in FIG. 9c, it was confirmed that the purified encapsulin (Encapsulin) protein formed VLP.

As a result of the above obtained results shown in FIG. 9c, it was confirmed that the purified encapsulin protein formed VLP. The diameter of the confirmed VLP appeared to be between 30 and 40 nm, which is the diameter of the existing encapsulin protein, and it was confirmed that a spherical VLP was formed even in the form of a fusion protein in which an antigen protein was fused to the C-terminus. In the results of Example 3, similar to the results of Example 2, both of high-efficiency protein production and VLP formation of encapsulin in *E. coli* by using the present invention were confirmed, and this indicates that an RID, which is a high-efficiency and water-solubility enhancing fusion partner of this experiment, is essential for forming a fusion protein-based platform in which proteins such as antigens are inserted at the C-terminus of encapsulin.

### [Example 4]

### Preparation of encapsulin corona virus antigen complex with N-terminal expression level and water solubility enhancement partner added for high efficiency and water solubility enhancement

### <Example 4-1>

In order to make encapsulin-based virus-like particles composed of an encapsulin protein fusion protein with a coronavirus RBD antigen (223a.a, 25.0 kDa) attached to the C-terminus, RBD (RNA Binding Domain), which is a coronavirus antigen was inserted. For the inserted coronavirus, the RBD (BetaCoV/Korea/KCDC03/2020, (NCBI access number: MN908947)) gene derived from the 2019-nCoV spike S protein was used (SEQ ID NO: 10). RBD, which is the antigen of the coronavirus, consists of 223 amino acids, and the molecular weight of the protein corresponds to about 25 kDa. Based on the protein sequence, the synthesized gene sequence was prepared after codon optimization in *E. coli* (SEQ ID NO: 10) (the gene synthesis was requested at BIONICS Co., Ltd.).

In FIG. 11, MSAVKAA (SEQ ID NO: 12), an RID peptide (SEQ ID NO: 13) and a mutant RID peptide with 50% or more similarity (SEQ ID NO: 14) were fused to prepare a recombinant protein, and the water-soluble expression pattern was verified.

In addition, a peptide having the amino acid sequence of (GaSb)n (a≥1, b≥1, n≥1) was used as a linker of the optimized sequence by applying ENLYFQG/S, which is the sequence of the TEV cleavage site, and it was placed between the TEV cleavage sequence and the fusion protein sequence. A mimetic diagram of the fusion protein expression vector constructed in this example is shown in FIG. 10b.

The gene of the above sequence was prepared based on the pET9a-Encapsulin vector, which is the recombinant complex protein expression vector prepared in Example 1, by using the pET-9a (Novagene, 69431-3CN) (SEQ ID NO: 11) expression vector. T7 RNA polymerase is expressed by IPTG, through which the RID sequence for increasing the water solubility and expression level was added to the N-terminus of the existing encapsulin complex protein vector sequence which was inserted into the MCS (Multiple Cloning Site) of the pET vector in which the Lac operon and T7 promoter present on the DE3 genome operate, and a linker sequence was added to enhance the TEV cleavage efficiency, and the pET9a vector was cut out by using Nde1, KpnI and BamHI cleavage enzymes, subcloned and reinserted. Among the cleavage enzyme sites present inside the MCS, RID and linker (Linker1) of this study, the cleavage enzyme site and the linker (Linker2) for increasing the cleavage efficiency of the TEV enzyme were inserted between the NdeI and KpnI sequences, and DNA sequences designed to have a protein in the form where encapsulin and the rotavirus antigen protein were fused were respectively inserted between the KpnI and BamHI cutting enzymes. In addition, each gene was linked to the pET vector and the inserted gene sequence through T4 DNA ligase, and it was confirmed through DNA sequence analysis that the bacterial clones generated by the ligation reaction of these DNAs had a codon-optimized nucleotide sequence inserted therein.

The sequence of the recombinant complex expression vector prepared in Example 4-1 is as follows.

MSA VKAA - RID-Linker 1- TEV-Linker2-ENC- Linker3 (3 -2)-Co V RBD [(SEQ ID NO: 12)-(SEQ ID NO: 14)-(SEQ ID NO: 15)-TEV-(SEQ ID NO: 16)-(SEQ ID NO: 1)-(SEQ ID NO: 18)-(SEQ ID NO: 10)]

:MSAVKAA-
AAVQAAEVKVDGSEPKLSANELAARLAAEAAVAEAEAAQAELSEKQLSQAT
AAATNHTTDNGVGPEEESVGT-HHHHHH-ENLYFQ-GSGSGS-
EFLKRSFAPLTEKQWQEIDNRAREIFKTQLYGRKFVDVEGPYGWEYAAHPLG
EVEVLSDENEVVKWGLRKSLPLIELRATFTLDLWELDNLERGKPNVDLSSLE
ETVRKVAEFEDEVIFRGCEKSGVKGLLSFEERKIECGSTPKDLLEAIVRALSIF
SKDGIEGPYTLVINTDRWINFLKEEAGHYPLEKRVEECLRGGKIITTPRIEDAL
VVSERGGDFKLILGQDLSIGYEDREKDAVRLFITETFTFQVVNPEALILLKF-
GGGSGGGSEAAAKGGGS-
RVQPTESIVRFPNITNLCPFGEVFNATRFASVYAWNRKRISNCVADYSVLYNSA
SFSTFKCYGVSPTKLNDLCFTNVYADSFVIRGDEVRQIAPGQTGKIADYNYKL
PDDFTGCVIAWNSNNLDSKVGGNYNYLYRLFRKSNLKPFERDISTEIYQAGS
TPCNGVEGFNCYFPLQSYGFQPTNGVGYQPYRVVVLSFELLHAPATVCGPKK
STNLVKNKCVNF

The prepared complex was named "RID-ENC-CoV RBD."

### <Example 4-2>

The expression vector prepared in Example 4-1 was transformed into HMS174 competent cells (Novagen (RecA mutation in a K-12 strain, Cat: 69453-3)). All transformed *E. coli* were cultured in 3 mL of LB medium containing 50 µg/mL kanamycin at 37°C at 250 rpm for 5 to 7 hours, then 1 mL was transferred to 10 mL, diluted 10 times and then further cultured. After about 2 to 4 hours, when O.D600 reached 0.5 to 0.7, 0.4 mM IPTG (Biosesang, Cat#I1006, Lot#LB0C0340, cas#367-93-1) was added, and it was cultured for 17 to 19 hours at 16°C (up to 21 hours). For the culture medium, only the E. coli precipitated through centrifugation was obtained and stored frozen at -80°C. 0.3 mL of a lysis buffer solution (50 mM Tris-HCl (pH 7.5), 150 mM NaCl, 5% glycerol, 0.1% Triton X-100 and 10 mM imidazole) was added to the *E. coli* harvest corresponding to 3 mL of culture medium, and after sonicating, the whole lysate (T, Total lysate) was separated into a precipitate (P, Precipitant) and a supernatant (S, Soluble lysate) through centrifugation, and then, as a result of analyzing by SDS-PAGE, it was confirmed that the encapsulin-coronavirus antigen fusion protein, in which RID was added to the N-terminus, exhibited the high-efficiency water-soluble expression.

### <Example 4-3>

Cells obtained by culturing and inducing the expression of 500 mL of *E. coli* using the same culture method as in Example 4-2, in which the high-efficiency water-soluble expression was confirmed, were resuspended with 75 mL of a lysis buffer solution [Buffer A [50 mM Tris-HCl] (pH 7.9), 150 mM NaCl, 5% glycerol, 10 mM imidazole]. Cells in the cell resuspension were disrupted by using an ultrasonic cell disrupter + (sonication: pulse 15s/50s, 70% amplitude, 2 min 30 s, 2 times total). The lysed cells were centrifuged at 13,500 rpm at 4°C for 10 minutes, and the supernatant was collected and Ni+ affinity chromatography was performed by using AKTA (GE Healthcare). First, the HisTrap HP (GE Healthcare Life Sciences, Little Chalfont, Buckinghamshire, UK, 5 mL size) column was equilibrated with Buffer A (50 mM Tris-HCl (pH 7.9), 150 mM NaCl, 5% glycerol and 10 mM imidazole), and then, the cell lysis supernatant was loaded onto the equilibrated column at a flow rate of 1 mL/min. The RID-ENC-CoV RBD protein was eluted through incrementally injecting imidazone with a linear concentration gradient in the concentration range of 10 mM to 1M by using Buffer A and Buffer B (50 mM Tris-HCl (pH 7.9), 150 mM NaCl, 5% glycerol, and 1 M imidazole) containing 1M imidazole, and fractions of 2 mL each were obtained.

After confirming the separation pattern of the target protein for the eluate through SDS-PAGE, the eluate of the corresponding fraction was dialyzed with Dialysis Buffer (20 mM Tris-HCl (pH 7.9), 5% glycerol, 10 mM NaCl, 0.02% Tween). -20) at 9°C for 12 to 16 hours. In this case, after dialyzing first for 4 hours, it was cleaved between the N-terminal RID and the encapsulin-coronavirus antigen fusion protein by using the TEV cleaving enzyme for the remaining 8 to 12 hours.

In order to isolate and purify the target fusion protein from which the N-terminal fusion partner RID had been removed, the HisTrap HP (GE Healthcare Life Sciences, Little Chalfont, Buckinghamshire, UK, 5 mL size) column was used to equilibrate with the same Solution A (20 mM Tris-HCl (pH 7.9), 5% glycerol, 10 mM NaCl, 0.02% Tween-20) as the Dialysis buffer, and then it was loaded onto the equilibrated column at a flow rate of 1 mL/min. The ENC-CoV RBD target protein from which the N-terminal fusion partner RID had been removed was recovered in a form that was eluted without being adsorbed to the column.

In order to increase the final purity of the target protein, ion exchange chromatography was performed by using AKTA (GE Healthcare). After equilibrating the column (HiTrap Q FF, 1 mL size (GE Healthcare Life Sciences, Little Chalfont, Buckinghamshire, UK)) with Buffer A (20 mM Tris-HCl (pH 7.9), 10 mM NaCl, 5% glycerol, 0.02% Tween-20), it was loaded at a flow rate of 1 mL/min, and the target protein was eluted by NaCl injection having a linear concentration gradient of 10 mM to 1 M through Buffer A and Buffer B (20 mM Tris-HCl (pH 7.9), 1 M NaCl, 5% glycerol, 0.02% Tween-20), and fractions of 1 mL each were obtained. As a result, the purification aspect and purity thereof are illustrated in FIG. 12f (refer to FIG. 12f). The purified ENC-CoV RBD protein was stored at 4°C in a state of containing 20% glycerol, and the concentration of the finally purified protein was quantified by using BSA (Amresco, Solon, OH, USA).

### <Example 4-4>

In order to confirm the overall diameter distribution of the fusion protein (VLP) composed of the ENC-CoV RBD fusion protein of Example 4-3, analysis was performed through dynamic light scattering (DLS). Analysis of the complex was measured by placing 1 mL of the sample in a cuvette at a temperature of 16°C using DLS (Particular Systems, Zetasizer Nano Family). Dynamic light scattering analysis of cells was confirmed by using the total intensity and mass for each area in the image. As shown in FIG. 13a, it was confirmed to have a distribution of particle sizes with a diameter of 30 to 40 nm in the Hydrodynamic Radius of DLS.

### <Example 4-5>

The appearance of virus-like particles (VLPs) containing the purified encapsulin protein was observed with an electron microscope. The purified encapsulin VLP was first placed on a copper grid for 1 minute, then stained with 2% uranyl acetate for 1 minute, dried at room temperature for 10 minutes and then photographed by using a transmission electron microscope (TEM, Transmission electron microscope (120 kV)); Talos L120C, FEI, Czech). As shown in FIG. 13b, it was confirmed that the purified ENC-CoV RBD protein formed VLP. The diameter of the confirmed VLP appeared to be between 30 and 40 nm, which is the diameter of the existing encapsulin protein, and it was confirmed that a spherical VLP was formed even in the form of a fusion protein in which the antigen protein was fused to the C-terminus.

Through the results of Example 4, when the VLP in which encapsulin and the target protein were fused according to the present invention was administered, it was confirmed that a high level of the target protein-specific antibody was induced, and this antibody had the excellent performance to inhibit the binding between the target protein and its receptor.

### [Example 5]

### Preparation of encapsulin dengue virus antigen complex with N-terminal expression level and water solubility enhancing partner peptide added for high efficiency and water solubility enhancement

### <Example 5-1>

A dengue virus antigen was used to construct an encapsulin-based virus-like particle composed of an encapsulin protein fusion protein with a target protein attached to the C-terminus. The RNA binding domain (RBD) was inserted. EDIII, which is a dengue virus antigen, was inserted as an encapsulin protein, and the EDIII (Dengue virus 2 isolate DENV-2/KBPV-VR-29, complete genome, Sequence ID: KP406804.1) gene derived from dengue virus was used (SEQ ID NO: 6). EDIII, which is an antigen of dengue virus, consists of 101 amino acids and has a size of about 11.4 kDa. Based on the protein sequence, a gene sequence synthesized after codon optimization in A. *coli* was prepared (SEQ ID NO: 6) (the gene synthesis was requested at BIONICS Co., Ltd.)

In FIG. 9, MSAVKAA (SEQ ID NO: 12), an RID peptide (SEQ ID NO: 13) and a mutant RID peptide with 50% or more similarity (SEQ ID NO: 14), which have the effects of increasing the expression level and increasing the water solubility, were fused at the N-terminus of the encapsulin protein, to prepare a recombinant protein, and the water-soluble expression pattern was verified. In the case of the mutant RID, in order to increase water solubility, the amino acids of RID were used by substituting lysine (Lys, K) with alanine (Ala, A) by one or more and nine or less.

In addition, a peptide having the amino acid sequence of (GaSb)n (a≥1, b≥1, n≥1) was used as a linker of the optimized sequence by applying ENLYFQG/S, which is the sequence of the TEV cleavage site, and this was placed between the TEV cleavage sequence and the fusion protein sequence. A mimetic diagram of the fusion protein expression vector constructed in this example is shown in FIG. 14b.

The gene of the above sequence was constructed based on the pET9a-ENC-Linker3(3-2)-CoV RBD vector and the pET9a-MSAVKAA-RID-Linker1-6xHIS-TEV-Linker2-ENC- Linker3(3-2)-CoV RBD vector, which are the recombinant complex protein expression vectors constructed in Example 2, by using the PET-9a (Novagene, 69431-3CN) (SEQ ID NO: 11) expression vector, and it was inserted by replacing the C-terminal coronavirus antigen protein of ENC with a dengue virus antigen by using the Notl and BamHI cleavage enzymes.

The sequence of the recombinant complex expression vector prepared in Example 3-1 is as follows.

MSA VKAA-RID-Linker1- TEV-Linker2-ENC-Linker3(3-2)-DENV EDIII [(SEQ ID NO:12)-(SEQ ID NO:13)-(SEQ ID NO:(SEQ ID NO:16)-(SEQ ID NO: 1)-(SEQ ID NO: 18)-(SEQ ID NO: 6)]

MSAVKAA-
AAVQAAEVKVDGSEPKLSKNELKRRLKAEKKVAEKEAKQKELSEKQLSQAT
AAATNHTTDNGVGPEEESV-GTHHHHHHENLYFQ-GSGSGS-
EFLKRSFAPLTEKQWQEIDNRAREIFKTQLYGRKFVDVEGPYGWEYAAHPLG
EVEVLSDENEVVKWGLRKSLPLIELRATFTLDLWELDNLERGKPNVDLSSLE
ETVRKVAEFEDEVIFRGCEKSGVKGLLSFEERKIECGSTPKDLLEAIVRALSIF
SKDGIEGPYTLVINTDRWINFLKEEAGHYPLEKRVEECLRGGKIITTPRIEDAL
VVSERGGDFKLILGQDLSIGYEDREKDAVRLFITETFTFQVVNPEALILLKF-
GGGSGGGSEAAAKGGGS-
KGMSYSMCTGKFKVVKEIAETQHGTIVIRVQYEGDGSPCKIPFEIMDLEKRH
VLGRLITVNPIVTEKDRPVNIEAEPPFGDSYIIIGVEPGQLKLNWFKKG

The prepared complex was named "RID-ENC-DENV EDIII."

### <Example 5-2>

The expression vector constructed in Example 5-1 was transformed into HMS174 competent cells (Novagen (RecA mutation in a K-12 strain, Cat: 69453-3)). All transformed *E. coli* were cultured in 3 mL of LB medium containing 50 µg/mL kanamycin at 37°C at 250 rpm for 5 to 7 hours, and then, 1 mL was transferred to 10 ml, diluted 10 times and then further cultured. After about 2 to 4 hours, when O.D600 reached 0.5 to 0.7, 0.4 mM IPTG (Biosesang, Cat#I1006, Lot#LB0C0340, cas#367-93-1) was added, and it was cultured for 17 to 19 hours at 16°C (up to 21 hours). For the culture medium, only the *E. coli* precipitated through centrifugation was obtained and stored frozen at -80°C. 0.3 mL of a lysis buffer solution (50 mM Tris-HCl (pH 7.5), 150 mM NaCl, 5% glycerol, 0.1% Triton X-100 and 10 mM imidazole) was added to the *E. coli* harvest corresponding to 3 mL of the culture medium, and after sonicating, the total lysate (T, Total lysate) was separated into a precipitate (P, Precipitant) and a supernatant (S, Soluble lysate) through centrifugation, and then analyzed by SDS-PAGE. As a result, as shown in FIG. 9, it was confirmed that the encapsulin-dengue virus antigen fusion protein in which the RID was added to the N-terminus showed the high-efficiency water-soluble expression.

### <Example 3-3>

Cells obtained by culturing and inducing the expression of 500 mL of *E. coli* using the same culture method as in Example 3-2, in which the high-efficiency water-soluble expression was confirmed, were resuspended with 75 mL of a lysis buffer solution [Buffer A [50 mM Tris-HCl] (pH 7.9), 150 mM NaCl, 5% glycerol, 10 mM imidazole]. Cells in the cell resuspension were disrupted by using an ultrasonic cell disrupter + (sonication: pulse 15s/50s, 70% amplitude, 2 min 30 s, 2 times total). The lysed cells were centrifuged at 13,500 rpm at 4°C for 10 minutes, and the supernatant was collected and Ni+ affinity chromatography was performed by using AKTA (GE Healthcare). First, the HisTrap HP (GE Healthcare Life Sciences, Little Chalfont, Buckinghamshire, UK, 5 mL size) column was equilibrated with Buffer A (50 mM Tris-HCl (pH 7.9), 150 mM NaCl, 5% glycerol and 10 mM imidazole), and then, the cell lysis supernatant was loaded onto the equilibrated column at a flow rate of 1 mL/min. The RID-ENC-DENV EDII protein was eluted through incrementally injecting imidazone with a linear concentration gradient in the concentration range of 10 mM to 1M by using Buffer A and Buffer B (50 mM Tris-HCl (pH 7.9), 150 mM NaCl, 5% glycerol, and 1 M imidazole) containing 1M imidazole, and fractions of 2 mL each were obtained.

After confirming the separation pattern of the target protein for the eluate through SDS-PAGE, the eluate of the corresponding fraction was dialyzed with Dialysis Buffer (20 mM Tris-HCl (pH 7.9), 5% glycerol, 10 mMNaCl, 0.02% Tween). -20) at 9°C for 12 to 16 hours. In this case, after dialyzing first for 4 hours, it was cleaved between the N-terminal RID and the encapsulin-dengue virus antigen fusion protein by using the TEV cleaving enzyme for the remaining 8 to 12 hours.

In order to isolate and purify the target fusion protein from which the N-terminal fusion partner RID had been removed, the HisTrap HP (GE Healthcare Life Sciences, Little Chalfont, Buckinghamshire, UK, 5 mL size) column was used to equilibrate with the same Solution A (20 mM Tris-HCl (pH 7.9), 5% glycerol, 10 mM NaCl, 0.02% Tween-20) as the Dialysis buffer, and then it was loaded onto the equilibrated column at a flow rate of 1 mL/min. The ENC-DENV EDII target protein from which the N-terminal fusion partner RID had been removed was recovered in a form that was eluted without being adsorbed to the column.

In order to increase the final purity of the target protein, ion exchange chromatography was performed by using AKTA (GE Healthcare). After equilibrating the column (Fractogel TMAE, 5 mL size (#125069, Merck)) with Buffer A (20 mM Tris-HCl (pH 7.9), 10 mM NaCl, 5% glycerol, 0.02% Tween-20), it was loaded at a flow rate of 1 mL/min, and the target protein was eluted by NaCl injection having a linear concentration gradient of 10 mM to 1 M through Buffer A and Buffer B (20 mM Tris-HCl (pH 7.9), 1 M NaCl, 5% glycerol, 0.02% Tween-20), and fractions of 1 mL each were obtained. As a result, the purification aspect and purity thereof are illustrated in FIG. 16f (refer to FIG. 16f). The purified ENC-DENV protein was stored at 4°C in a state of containing 20% glycerol, and the concentration of the finally purified protein was quantified by using BSA (Amresco, Solon, OH, USA).

### <Example 5-4>

In order to confirm the overall diameter distribution of the fusion protein (VLP) composed of the ENC-DENV EDIII fusion protein of Example 5-3, analysis was performed through dynamic light scattering (DLS). Analysis of the complex was measured by placing 1 mL of the sample in a cuvette at a temperature of 16°C using DLS (Particular Systems, Zetasizer Nano Family). Dynamic light scattering analysis of cells was confirmed by using the total intensity and mass for each area in the image. As shown in FIG. 17a, it was confirmed to have a distribution of particle sizes with a diameter of 30 to 40 nm in the Hydrodynamic Radius of DLS.

### <Example 5-5>

The appearance of virus-like particles (VLPs) containing the purified encapsulin protein was observed with an electron microscope. The purified encapsulin VLP was first placed on a copper grid for 1 minute, then stained with 2% uranyl acetate for 1 minute, dried at room temperature for 10 minutes and then photographed by using a transmission electron microscope (TEM, Transmission electron microscope (120 kV)); Talos L120C, FEI, Czech). As shown in FIG. 11b, it was confirmed that the purified ENC-DENV protein formed VLP. The diameter of the confirmed VLP appeared to be between 30 and 40 nm, which is the diameter of the existing encapsulin protein, and it was confirmed that a spherical VLP was formed even in the form of the fusion protein in which the antigen protein was fused to the C-terminus.

In the results of Example 4, similar to the results of Example 2, both high-efficiency protein production and VLP formation in the *E. coli* of encapsulin using the present invention were confirmed, and this indicates that RID, which is a high-efficiency and water-solubility enhancing fusion partner of this experiment, is essential for forming a fusion protein-based platform in which proteins such as antigens are inserted at the C-terminus of encapsulin.

Although the exemplary embodiments of the present invention have been described above, those skilled in the art will understand that the present invention may be implemented in other specific forms without changing the technical spirit or essential features. Therefore, it should be understood that the exemplary embodiments described above are illustrative in all respects and not restrictive.

## Claims

1. A recombination expression vector for vaccine production, comprising polynucleotides that encode:
a target protein;
an encapsulin protein; and
an RNA interacting domain (RID) protein.

2. The recombination vector of claim 1, wherein the encapsulin protein comprises the amino acid sequence of SEQ ID NO: 1 or a partial sequence thereof.

3. The recombinant vector of claim 1, wherein the RID protein is a fusion partner for increasing an expression level or water solubility of the vaccine.

4. The recombinant vector of claim 1, wherein the RID protein comprises the amino acid sequence of SEQ ID NO: 12, SEQ ID NO: 13 or SEQ ID NO: 14 or a partial sequence thereof.

5. The recombinant vector of claim 1, wherein at least one linker protein is located between the target protein, the encapsulin protein and the RNA interacting domain (RID) protein, and
wherein the linker protein comprises the gene sequence of SEQ ID NO: 15, SEQ ID NO: 16, SEQ ID NO: 17 or SEQ ID NO: 18.

6. The recombinant expression vector of claim 1, wherein the polynucleotide further encodes a TEV protein for cleavage of the RID protein.

7. The recombinant expression vector of claim 1, wherein the target protein is any one protein selected from the group consisting of a viral antigen protein, an antibody, a cell receptor, an enzyme, a structural protein, a serum and a cell protein.

8. The recombinant expression vector of claim 7, wherein the viral antigen protein is any one protein selected from the group consisting of a rotavirus antigen protein, a cervical cancer virus antigen protein, a dengue virus antigen protein and a coronavirus antigen protein.

9. A host cell transformed by the recombinant expression vector according to claim 1.

10. The transformed host cell of claim 9, wherein the host cell is *E. coli.*

11. A fusion protein, comprising:
a target protein; an encapsulin protein; and an RNA interacting domain (RID) protein.

12. The fusion protein of claim 11, wherein the target protein is linked to the C-terminus of the encapsulin protein.

13. The fusion protein of claim 11, wherein at least one linker protein is located between the target protein, the encapsulin protein and the RNA interacting domain (RID) protein, and
wherein the linker protein comprises the gene sequence of SEQ ID NO: 15, SEQ ID NO: 16, SEQ ID NO: 17 or SEQ ID NO: 18.

14. A method for producing a vaccine, comprising the steps of:
a) producing a recombinant expression vector for vaccine production comprising polynucleotides that encode a target protein; an encapsulin protein; and an RID protein;
b) producing a transformant by introducing the recombinant expression vector into a host cell; and
c) culturing the transformant to induce the expression of a recombinant fusion protein, and obtaining the same.

15. The method of claim 14, wherein the RID protein is a fusion partner for increasing an expression level or water solubility of the vaccine.

16. The method of claim 14, wherein the RID protein comprises the amino acid sequence of SEQ ID NO: 12, SEQ ID NO: 13 or SEQ ID NO: 14 or a partial sequence thereof.

17. The method of claim 14, wherein the host cell is *E. coli.*

18. The method of claim 14, wherein the encapsulin protein comprises the amino acid sequence of SEQ ID NO: 1 or a partial sequence thereof.

19. The method of claim 14, wherein the target protein is any one protein selected from the group consisting of a viral antigen protein, an antibody, a cell receptor, enzyme, a structural protein, a serum and a cell protein.

20. The method of claim 19, wherein the viral antigen protein is any one protein selected from the group consisting of a rotavirus antigen protein, a cervical cancer virus antigen protein, a dengue virus antigen protein and a coronavirus antigen protein.

21. The method of claim 14, wherein at least one linker protein is located between the target protein, the encapsulin protein and the RNA interacting domain (RID) protein, and
wherein the linker protein comprises the gene sequence of SEQ ID NO: 15, SEQ ID NO: 16, SEQ ID NO: 17 or SEQ ID NO: 18.
